# EUROPEAN PATENT SPECIFICATION

(11) **EP 4 225 347 B1**
(45) Date of publication and mention of the grant of the patent: **25.12.2024**
(21) Application number: 21783547.9
(22) Date of filing: 06.10.2021
(51) Int. Cl.: A61K 38/17, A61P 35/00

(54) **CONNEXIN 43 FOR USE IN THE TREATMENT OF A CANCER TYPE CHARACTERIZED BY THE ACTIVATION OF A MITOGEN-ACTIVATED PROTEIN KINASE**
CONNEXIN 43 ZUR VERWENDUNG IN DER BEHANDLUNG EINES KREBSTYPS, DER DURCH DIE AKTIVIERUNG EINER MITOGENAKTIVIERTEN PROTEINKINASE CHARAKTERISIERT IST
CONNEXINE 43 DESTINÉE À ÊTRE UTILISÉE DANS LE TRAITEMENT D'UN TYPE DE CANCER CARACTÉRISÉ PAR L'ACTIVATION D'UNE PROTÉINE KINASE ACTIVÉE PAR MITOGÈNE

(30) Priority: 06.10.2020 EP 20382883
(43) Date of publication of application: 16.08.2023
(73) Proprietor: Fundación Profesor Novoa Santos, 15006 A Coruña (ES); Servizo Galego de Saúde, 15703 Santiago de Compostela (ES)
(72) Inventor: MAYÁN SANTOS, María Dolores, 15006 A Coruña (ES); VARELA VÁZQUEZ, Adrián, 15006 A Coruña (ES); GUITIÁN CAAMAÑO, Amanda, 15006 A Coruña (ES); CARPINTERO FERNÁNDEZ, Paula, 15006 A coruña (ES); VARELA EIRÍN, Marta, 15006 A Courña (ES); FONSECA CAPDEVILA, Eduardo, 15703 Santiago de Compostela (ES); QUINDÓS VARELA, María, 15703 Santiago de Compostela (ES); CALLEJA CHUCLA, Teresa, 15703 Santiago de Compostela (ES)
(74) Representative: Hoffmann Eitle
(86) International application number: PCT/EP2021/077487
(87) International publication number: WO 2022/074025

(56) References cited:
- WO-A1-2017/147561
- DHILLON SOHITA: "Dabrafenib plus Trametinib: a Review in Advanced Melanoma with aBRAFV600Mutation", TARGETED ONCOLOGY, SPRINGER PARIS, PARIS, vol. 11, no. 3, 1 June 2016 (2016-06-01), pages 417 - 428, XP035975138, ISSN: 1776-2596, [retrieved on 20160601], DOI: 10.1007/S11523-016-0443-8
- KHUNGER ARJUN ET AL: "Dabrafenib in combination with trametinib in the treatment of patients with BRAF V600-positive advanced or metastatic non-small cell lung cancer: clinical evidence and experience", vol. 12, 1 January 2018 (2018-01-01), pages 1 - 9, XP055778160, Retrieved from the Internet <URL:https://www.ncbi.nlm.nih.gov/pmc/articles/PMC5941661/pdf/10.1177_1753466618767611.pdf>
- SULLIVAN RYAN J. ET AL: "Abstract LB-B30: First in human, dose escalation trial of the combination of dabrafenib, trametinib, and navitoclax in patients with BRAF mutant solid tumors", THERAPEUTIC AGENTS: SMALL-MOLECULE KINASE INHIBITORS, 1 January 2018 (2018-01-01), pages B - B30, XP055778174, DOI: 10.1158/1535-7163.TARG-17-LB-B30
- J. L. KIRKLAND ET AL: "Senolytic drugs: from discovery to translation", JOURNAL OF INTERNAL MEDICINE, vol. 288, no. 5, 4 August 2020 (2020-08-04), GB, pages 518 - 536, XP055746771, ISSN: 0954-6820, DOI: 10.1111/joim.13141
- A S DHILLON ET AL: "MAP kinase signalling pathways in cancer", ONCOGENE, vol. 26, no. 22, 14 May 2007 (2007-05-14), pages 3279 - 3290, XP055118487, ISSN: 0950-9232, DOI: 10.1038/sj.onc.1210421

## Description

### FIELD OF THE INVENTION

The present invention refers to the medical field. Particularly, the present invention refers to Connexin 43 (Cx43) for use in the treatment of cancer wherein the cancer is characterized by the activation of a mitogen-activated protein kinase (MAPK) selected from the list consisting of: BRAF, RAS, MEK or ERK. The present invention also refers to a combination drug product comprising Cx43 and an inhibitor of a mitogen-activated protein kinase (MAPK) selected from the list consisting of: BRAF, RAS, MEK or ERK.

### STATE OF THE ART

Mutations of MAPK, for instance BRAF mutations, are detected in many types of cancer. BRAF is a potent oncogene that is activated in more than 8 % of all cancer types. Approximately about 30 - 50 % of thyroid cancer, 3 % of non-small cell lung cancer (NSCLC), 5 % to 10 % of patients with metastatic colorectal cancer, 100 % of hairy cell leukaemia and more than 50 % of melanoma patients have a mutation of the proto-oncogene BRAF, that lead to a constitutive activation of the serine/threonine kinases of the MAPK cascade.

Mutations in BRAF were initially described in 2002, with V600E being the most common mutation. These mutations lead to constitutive activation of BRAF and RAS-RAF-MEK-ERK signalling cascade, which promotes cancer growth by enhancing cell proliferation and survival while inhibiting cell death by apoptosis. Following the discovery of V600E mutation, targeted therapies including BRAF-specific small molecule inhibitors such as dabrafenib, and later the MEK inhibitors such as trametinib, were developed for the treatment of metastatic disease. BRAF/MEK inhibitors (BRAF/MEKi) are indicated for patients with unresectable or metastatic melanoma due their efficacy, and the combination of these inhibitors has improved the progression-free survival. In contrast to patients with BRAF mutated metastatic melanoma, only 5% of patients with BRAF mutated colorectal cancer responded to BRAF inhibitors monotherapy. Efforts to define resistance mechanisms to BRAF inhibition have led to new clinical trials using combined therapies in order to avoid primary and acquired resistance to BRAF inhibitors.

As previously mentioned, targeting MAPK pathway with specific BRAF/MEKi showed high initial responses in the majority of mutant BRAF melanoma patients. However, more than 50% of patients acquire resistance to the drugs and, consequently, cancer relapse. Also, some patients with BRAF mutant melanomas do not respond to treatment because of intrinsic mechanisms of resistance.

As a result, there is an unmet medical need of finding new pharmaceutical strategies for reducing the resistance to MAPK inhibitors and, consequently, for ameliorating patient relapse.

The present invention solves this problem by using Cx43, preferably in combination with MAPK inhibitors.

### DESCRIPTION OF THE INVENTION

### Brief description of the invention

As explained above, the present invention refers to Cx43, preferably in combination with an inhibitor of a mitogen-activated protein kinase (MAPK) selected from the list consisting of: BRAF, RAS, MEK or ERK, for use in the treatment of cancer wherein the cancer is characterized by the activation of mitogen-activated protein kinase (MAPK) selected from the list consisting of: BRAF, RAS, MEK or ERK.

Kindly note that cancer types characterized by the activation of MAPK are well-established in the prior art, such as it is indicated for instance in this document: [A S Dhillon, et al., 2007. MAP kinase signalling pathways in cancer. Oncogene volume 26, pages 3279-3290(2007). Published: 14 May 2007*].*

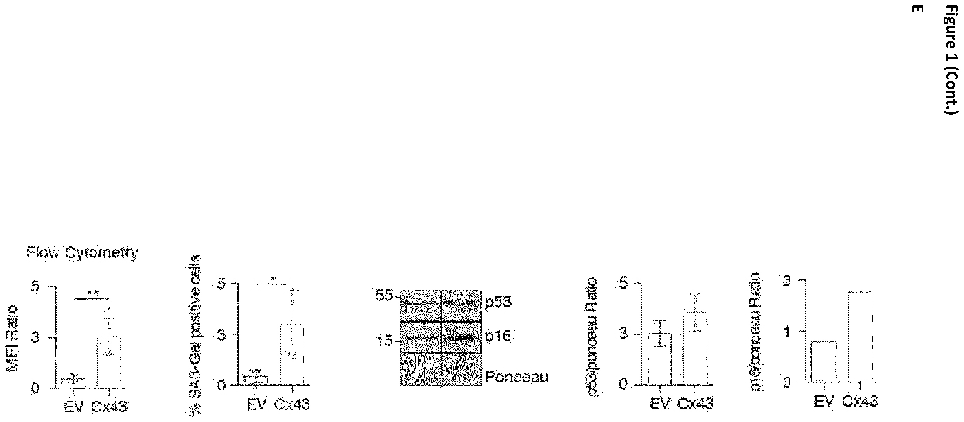

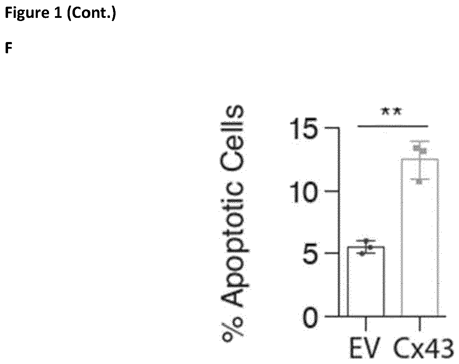

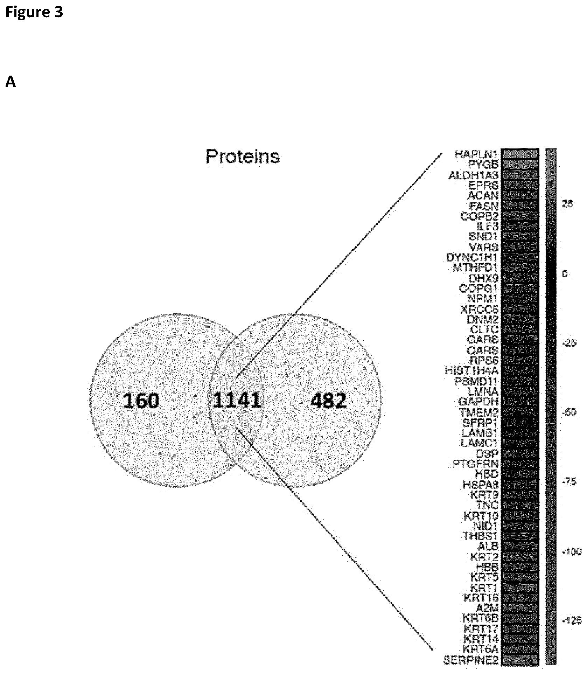

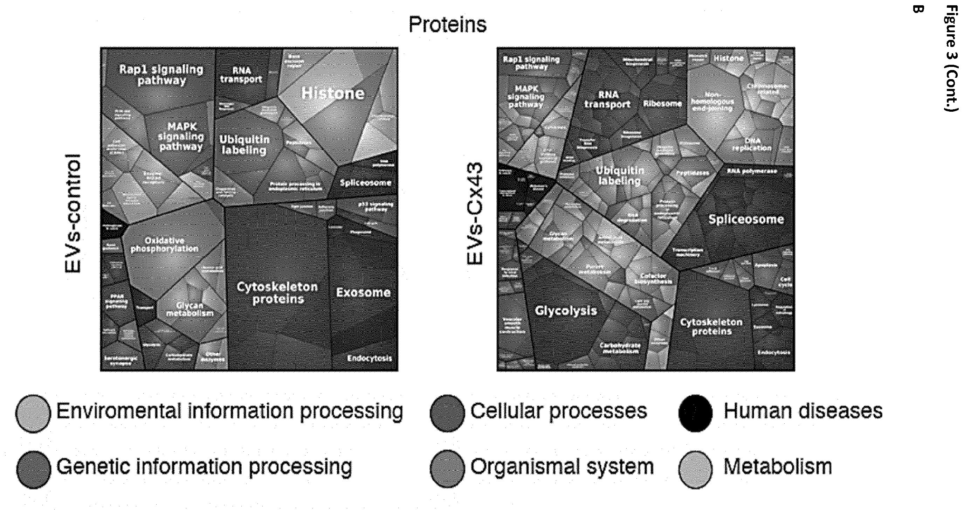

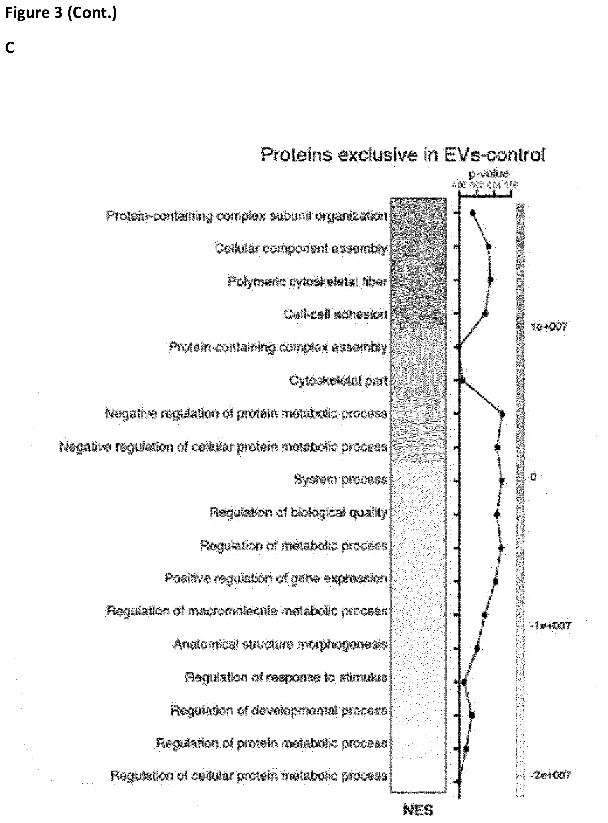

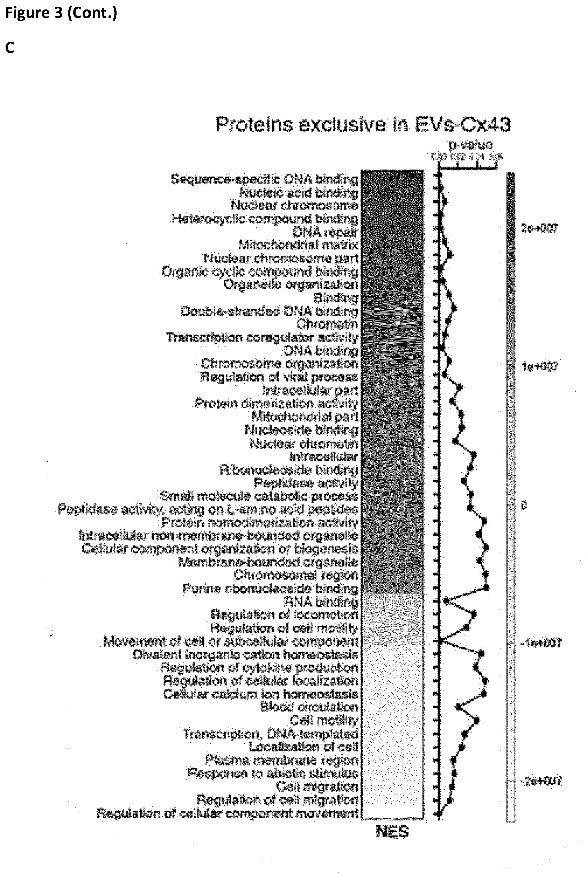

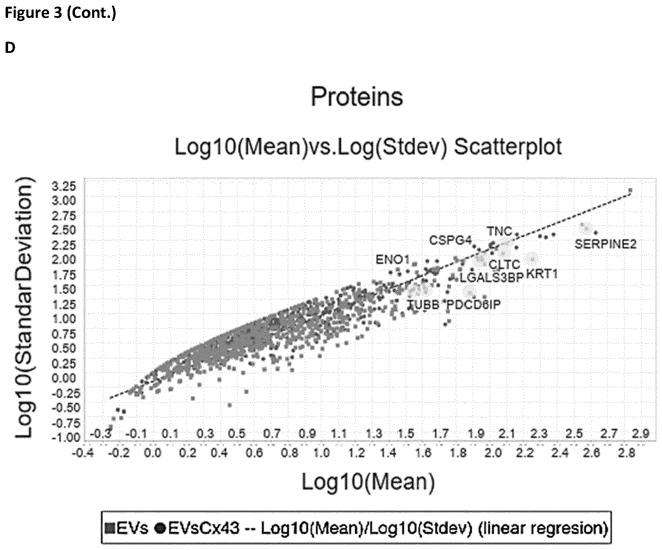

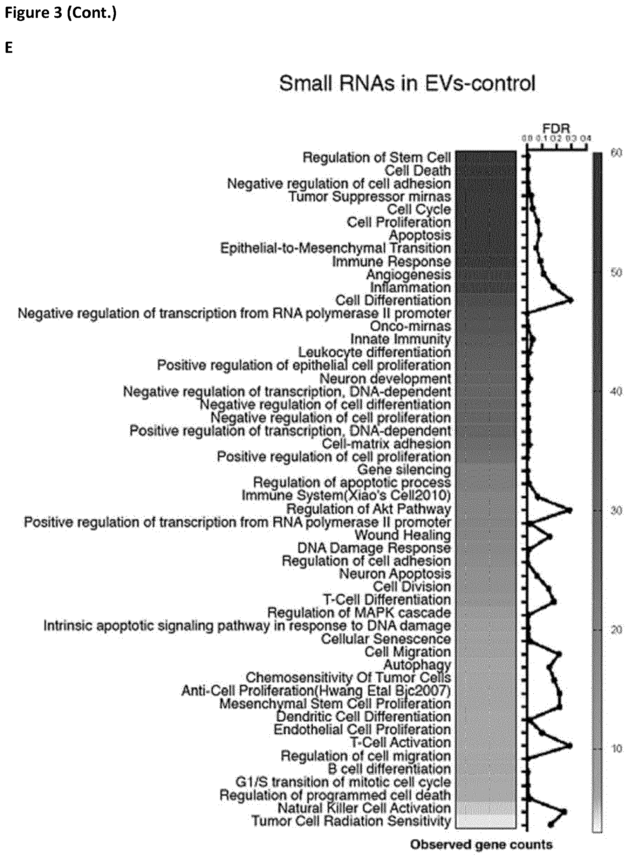

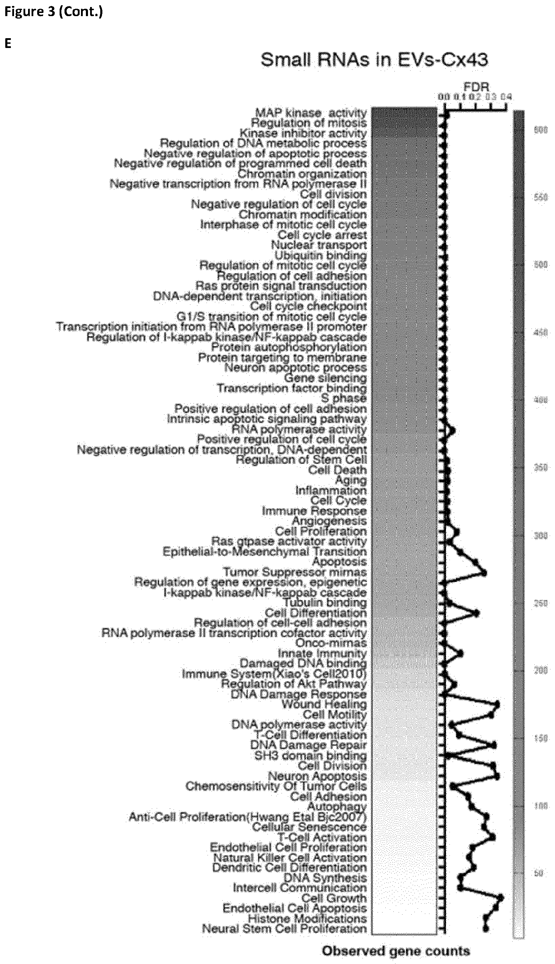

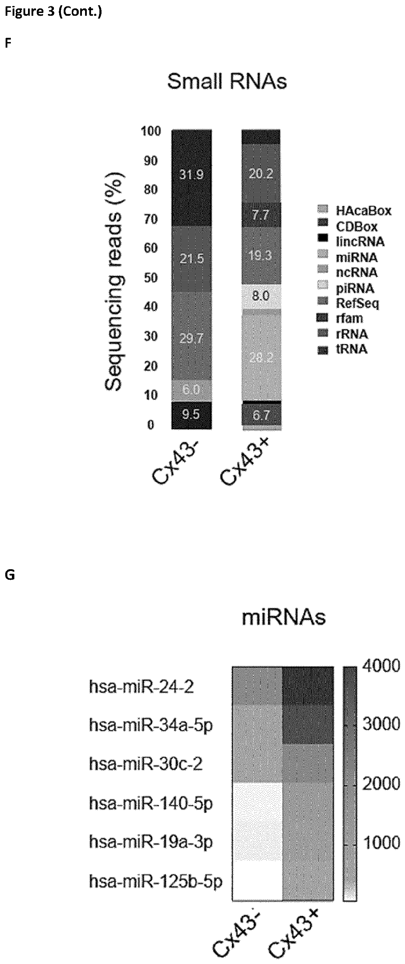

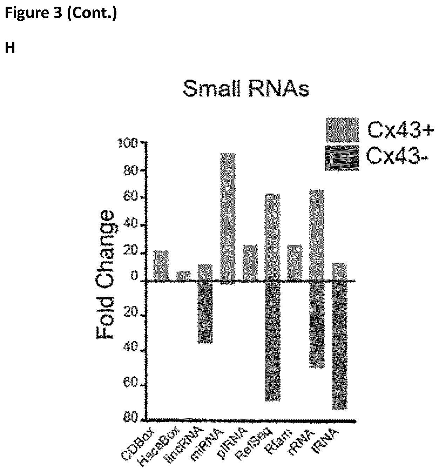

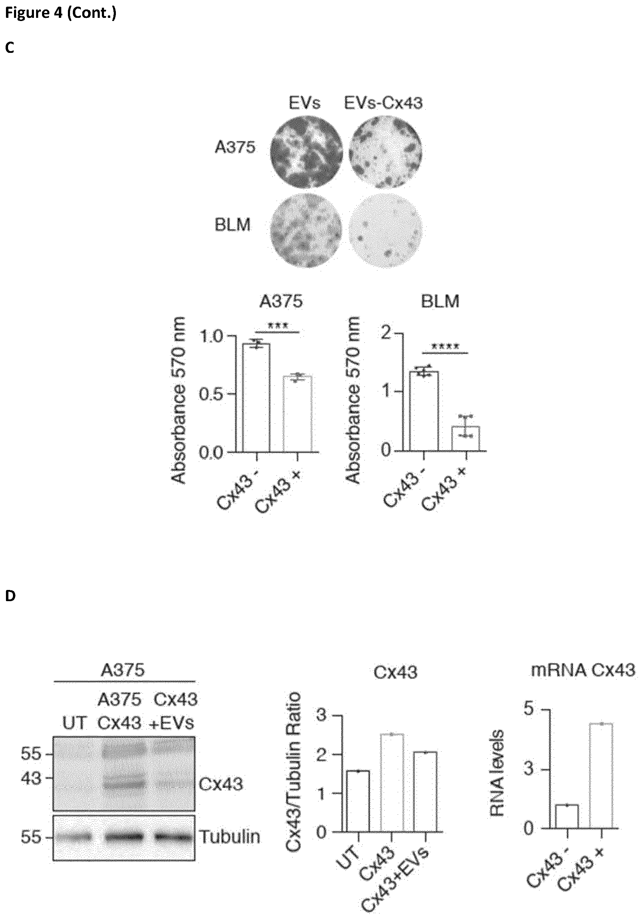

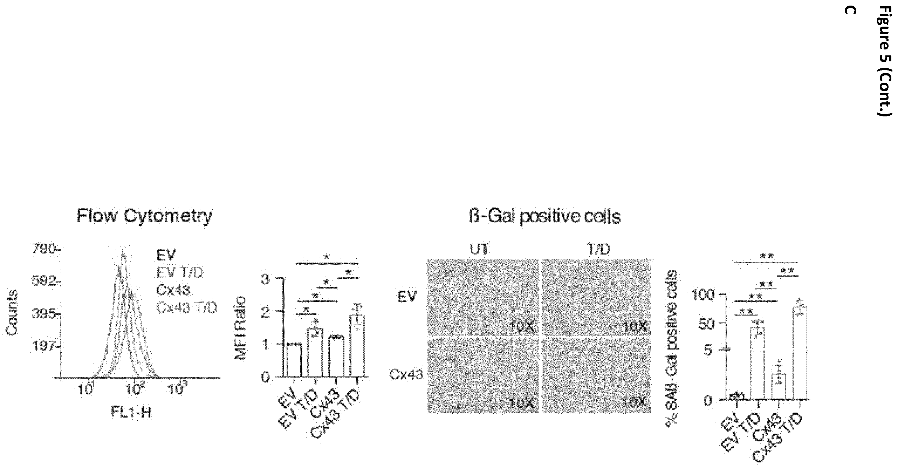

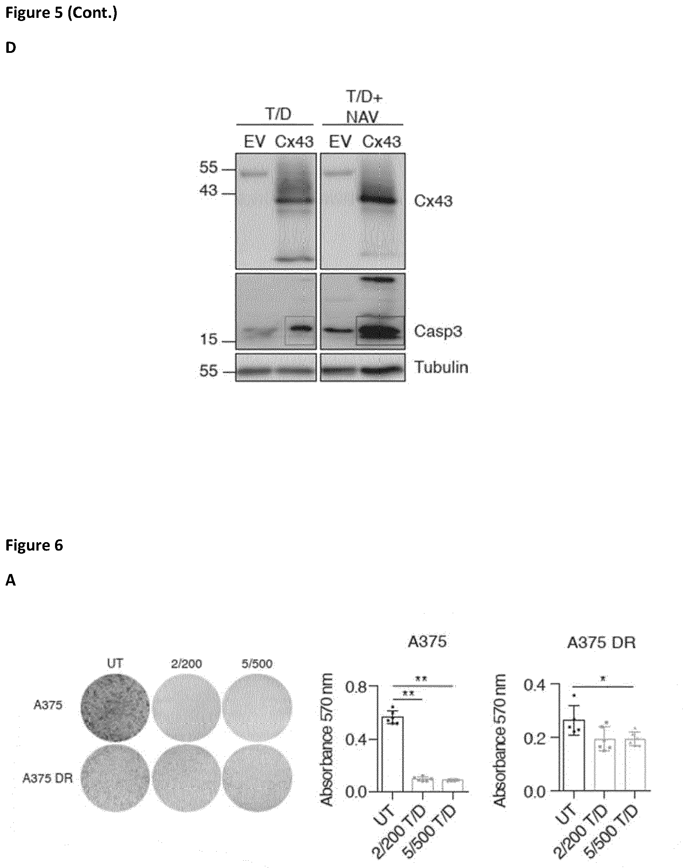

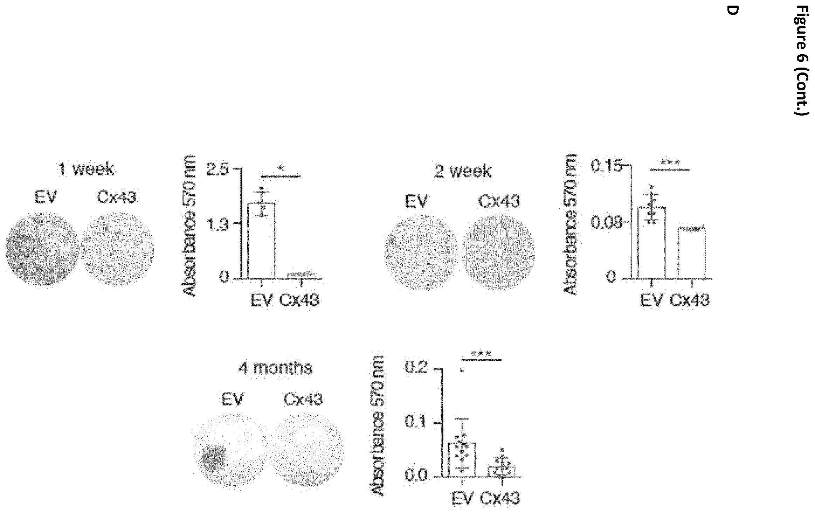

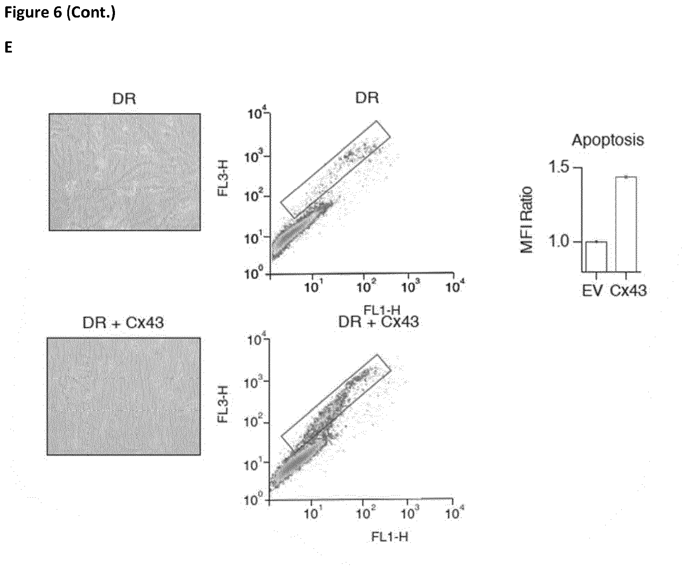

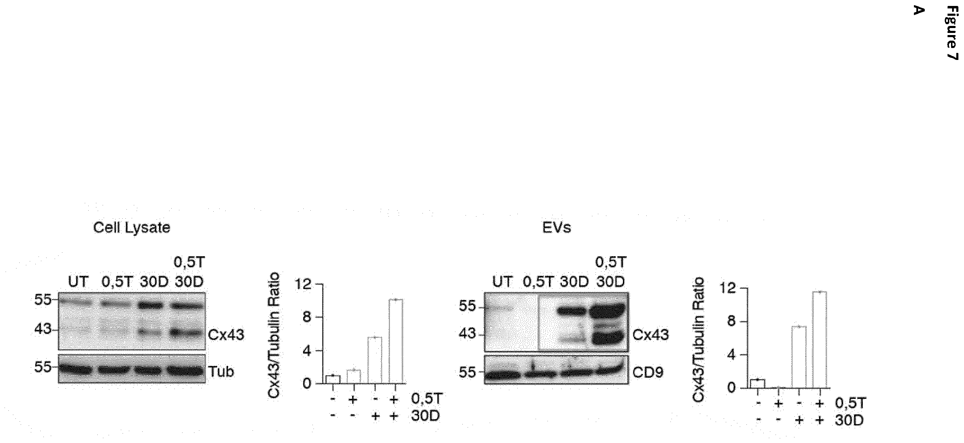

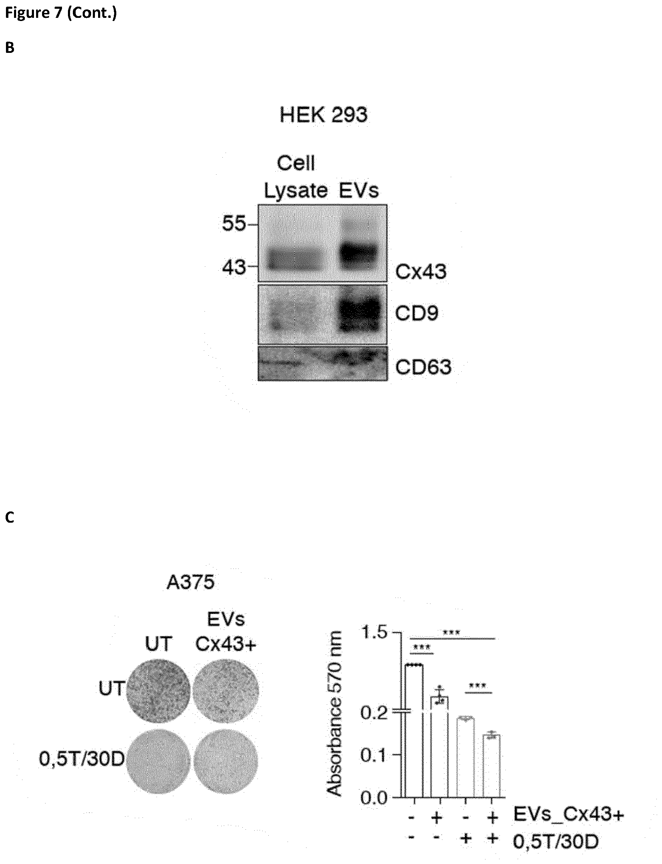

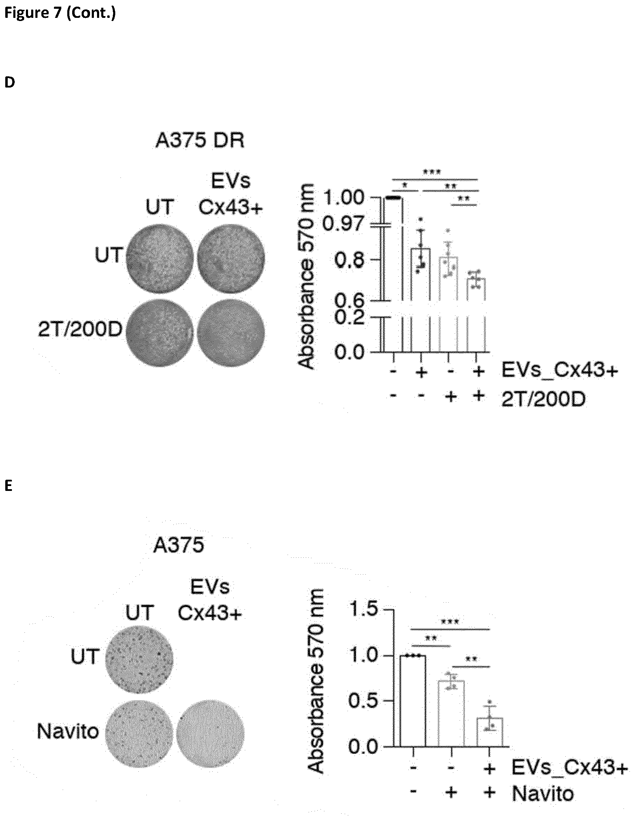

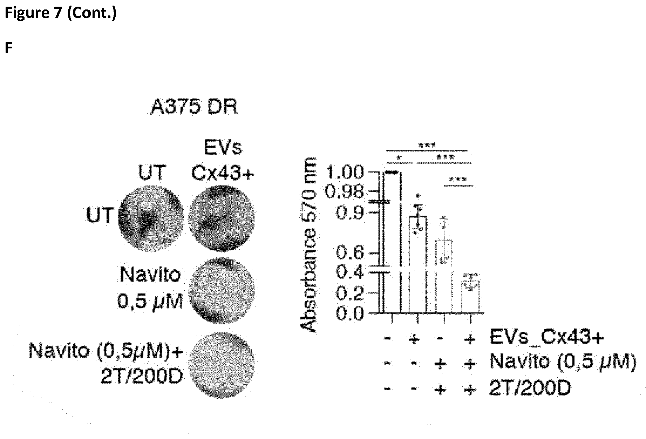

Particularly, the inventors of the present invention have found that Cx43 induces cellular senescence in BRAF-mutated tumour and enhances BRAF/MEK-induced senescence (**Figure 1e** and **Figure 5c**) and sensitizes cancer senescent cells to cell death by apoptosis mediated by activation of Caspase 3 (**Figures 1f** and **Figure 5d**). Reactivation of Cx43 avoids acquisition of drug resistance in tumour cells (**Figure 6d**) and more importantly, re-sensitized resistant tumour cells to BRAF/MEKi treatment by inducing cell death by apoptosis (**Figure 6e**). Further, the treatment with extracellular vesicles (EVs) enriched in Cx43, radically changed the small RNA and protein content (**Figure 3**) and switch the role of these tumour progression vehicles in target cells to tumour suppressors, reducing the capacity to proliferate and form colonies (**Figure 4c** and **Figure 7**). These EVs-containing Cx43 increase the efficacy of BRAF/MEKi in *in vitro* assays (Figure 7 c-d). The results provided in the present invention demonstrate a new role for Cx43 to avoid drug resistance, whose activation (using an expression vector or EVs) has the potential to improve the BRAF/MEKi therapy.

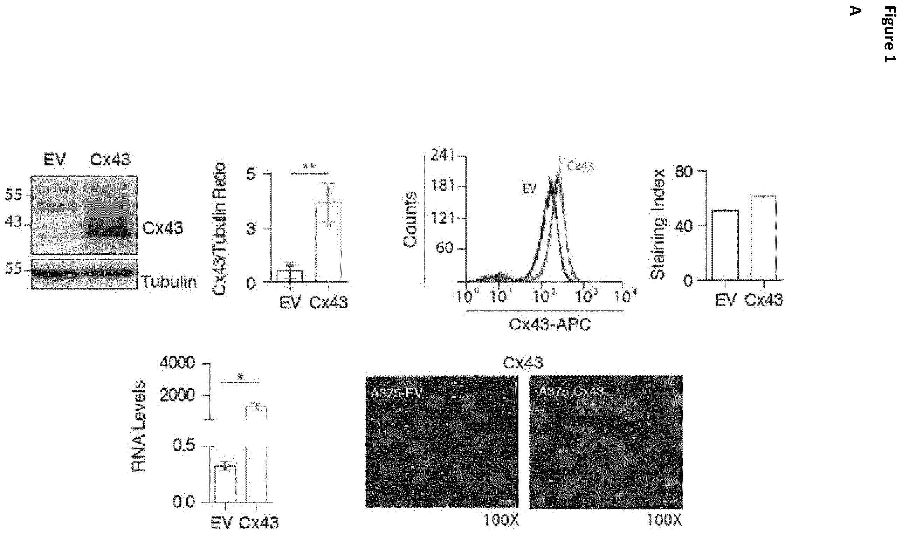

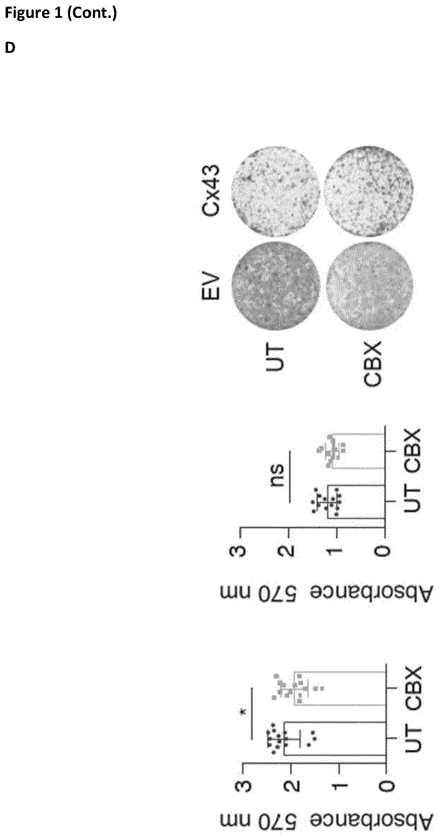

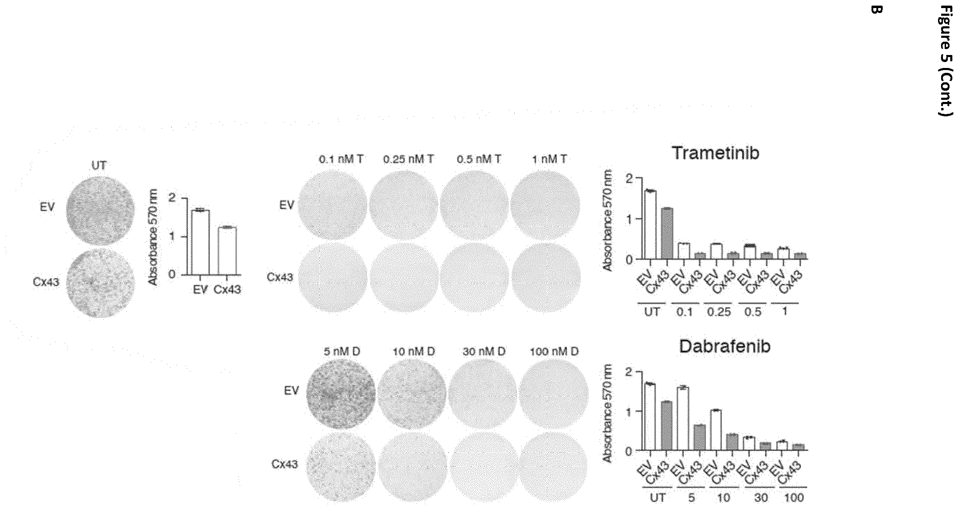

The channel protein Cx43, in a channel-independent manner (**Figure 1d**), induces cellular senescence (**Figure 1e**) and reactivate the pro-apoptotic effect of BRAF/MEKi in senescent cancer cells (**Figure 1f**). The results provided in the present invention indicate that the reactivation of Cx43 in BRAF mutant cells using a vector to overexpress Cx43 or EVs-containing Cx43 (**Figure 1a** and **Figure 2** **and** **Figure 7b**) increases BRAF/MEKi efficiency (**Figure 5b** and **Figure 7c****-d**) by reinforcing cellular senescence (**Figure 5c**) and reactivate the pro-apoptotic effect of BRAF/MEKi (**Figure 5d**). Further, when the senolytic drug navitoclax (ABT-263) was applied in parallel to BRAF/MEKi and in the presence of Cx43 (EVs containing Cx43, **Figure 7e****-f** or overexpressing vector, **Figure 5d**), significantly increased the efficacy to BRAF/MEKi in drug resistant tumour cells (**Figure 7f**), providing a rationale for combined therapeutics application in order to sensitize resistant metastatic cancer cells to therapy.

The restoration of Cx43, using a vector or via EVs, increases BRAF/MEKi efficiency, preventing and reverting drug resistance (**Figure 6d****-e** and **Figure 7d**). Navitoclax is a senolytic drug currently used in clinical trials in combination with the BRAF/MEKi dabrafenib and trametinib to improve their efficacy and to avoid drug resistance (ClinicalTrials.gov Identifier: NCT01989585). The results provided in the present invention demonstrate that EVs containing Cx43, or the reactivation of Cx43 using a vector (gene therapy), also improve the efficiency of BRAF/MEKi when those inhibitors are combined with navitoclax (**Figure 5d** and **Figure 7f**).

So, the first embodiment of the present invention refers to Cx43 for use in the treatment of cancer wherein the cancer is characterized by the activation of mitogen-activated protein kinase (MAPK) selected from the list consisting of: BRAF, RAS, MEK or ERK.

In a preferred embodiment, the cancer is characterized by the activation of BRAF or NRAS.

In a preferred embodiment, the cancer selected from: melanoma, colon cancer, lung cancer or breast cancer.

In a preferred embodiment, Cx43 is administered before, after or simultaneously to a treatment with an inhibitor of a mitogen-activated protein kinase (MAPK) selected from the list consisting of: BRAF, RAS, MEK or ERK.

In a preferred embodiment, Cx43 is administered before, after or simultaneously to a treatment with a BRAF and/or MEK inhibitor.

In a preferred embodiment, Cx43 is administered before, after or simultaneously to a treatment with dabrafenib, trametinib, vemurafenib, encorafenib, cobimetinib and/or binimetinib.

In a preferred embodiment, Cx43 is administered before, after or simultaneously to a treatment with a senolytic agent, preferably navitoclax.

The second embodiment refers to a combination drug product comprising Cx43 and an inhibitor of a mitogen-activated protein kinase (MAPK) selected from the list consisting of: BRAF, RAS, MEK or ERK.

In a preferred embodiment, the combination drug product comprises Cx43 and a BRAF and/or MEK inhibitor.

In a preferred embodiment, the combination drug product comprises Cx43 and dabrafenib, trametinib, vemurafenib, encorafenib, cobimetinib and/or binimetinib.

In a preferred embodiment, the combination drug product further comprises a senolytic agent, preferably navitoclax.

The third embodiment of the invention refers to a pharmaceutical composition comprising the combination drug product of the invention and, optionally, pharmaceutically acceptable excipients or carriers.

The fourth embodiment of the invention refers to the pharmaceutical composition of the invention for use in the treatment of cancer wherein the cancer is characterized by the activation of mitogen-activated protein kinase (MAPK) selected from the list consisting of: BRAF, RAS, MEK or ERK wherein Cx43 is administered by using a delivery vehicle.

In a preferred embodiment, the delivery vehicle is a nanoparticle, an extracellular vesicle or an expression vector which encodes Cx43. Particularly, any of the extracellular vesicles known in the prior art may be used for this purpose [Guillaume van Niel, et al., 2018. Shedding light on the cell biology of extracellular vesicles. Nature Reviews Molecular Cell Biology volume 19, pages 213-228(2018*)] [*Oscar P. B. Wiklander et al., 2019. Advances in therapeutic applications of extracellular vesicles. Science Translational Medicine. 15 May 2019. Vol. 11, Issue 492, eaav8521. DOI: 10.11261scitranslmedaav8521*].*

The last embodiment of the present invention refers to a method for treating cancer, wherein the cancer is characterized by the activation of mitogen-activated protein kinase (MAPK) selected from the list consisting of: BRAF, RAS, MEK or ERK, which comprises the administration of a therapeutically effective amount of Cx43, preferably in combination with any of the above defined mitogen-activated protein kinase (MAPK) inhibitors, and most preferably, also in combination with a senolytic agent (as defined above), or a pharmaceutical composition comprising thereof.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the cell suspension of the invention and that causes no significant adverse toxicological effects to the patient.
- By "therapeutically effective dose or amount" of a composition comprising the cell suspension of the invention is intended an amount that, when administered as described herein, brings about a positive therapeutic response in a subject having cancer. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

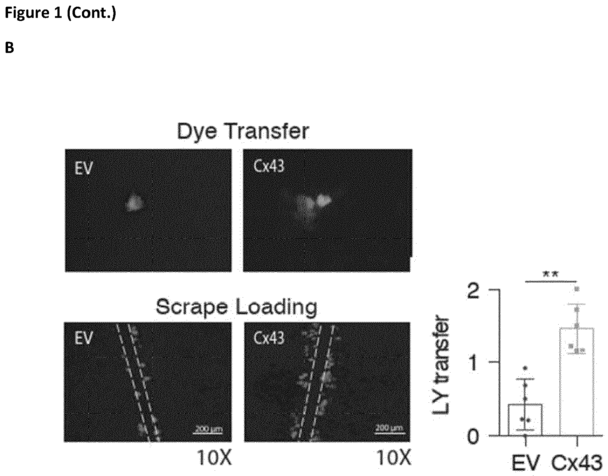

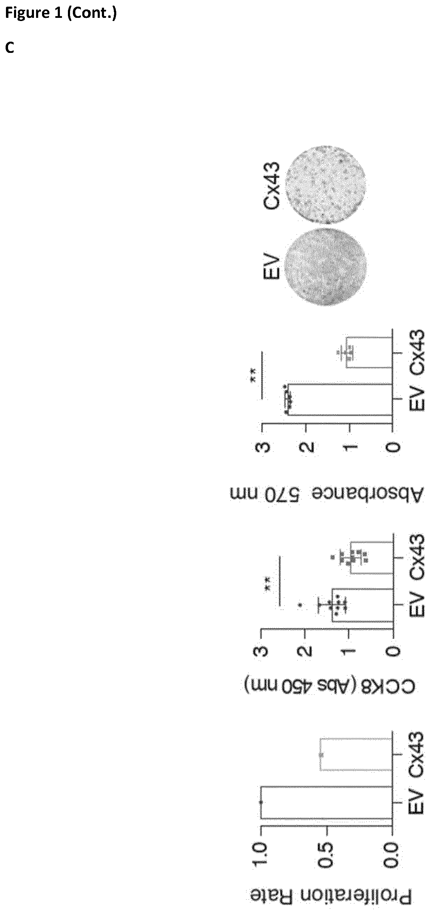

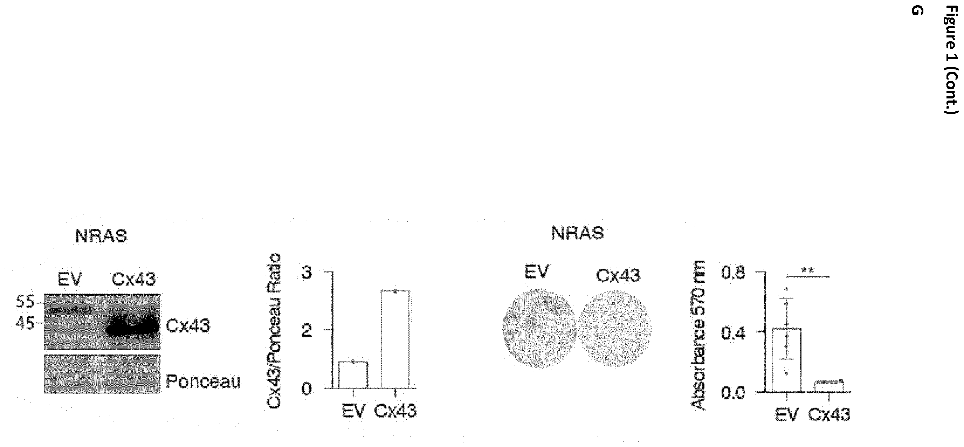

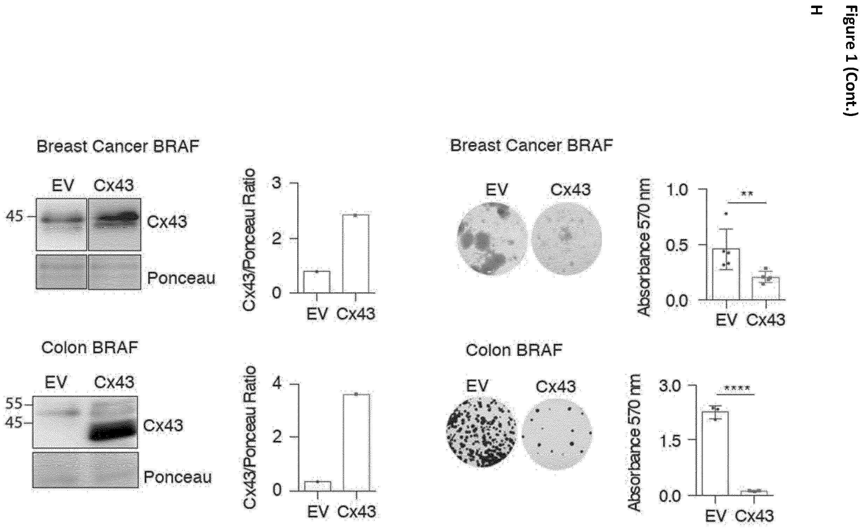

**Figure 1****. Restoration of Connexin 43 (Cx43) gene expression in cancer cells with a mutation in BRAF and NRAS, has anti-tumour effects. (a)** The transfection of the human melanoma cell line A375 with a mutation in BRAF with a vector to restore Cx43 gene expression leads to increased levels of Cx43 protein detected by western-blot (left, n=3) and flow cytometry (right, n=1). In the image, wild type cells (A375) transfected with an empty vector (EV, control) and transfected with the vector containing Cx43 gene under the human cytomegalovirus promoter (Cx43). Tubulin was used as loading control. Below: Cx43 mRNA levels detected by Real Time RT-qPCR (n=2) in EV and Cx43 transfected cells. Below, on the right: immunofluorescence assay to study Cx43 levels and subcellular localization in control tumour cells (A375) transfected with the EV (A375-EV) and with the Cx43 (A375-Cx43) gene expressing vector. Red arrows indicate positive spots for Cx43 in the margin of cells (membrane). **(b)** Scrape loading (bottom) and dye transfer (above) experiments were used to study gap junction intercellular communication (GJIC) in tumour cells, in absence (EV) or presence of Cx43 (Cx43) in the A375 cell line. The quantification of dye transference by scrape loading is shown on the right (n=6). **(c)** Cellular proliferation was tested using the xCELLigence system (n=1) and quantified by CCK-8 proliferation assay (n=3), as well as confirmed by colony formation assay (n=3). The presence of Cx43 restores GJIC (b) and leads to reduced capacity of colony formation and decreases the levels of proliferation. **(d)** No changes were detected in colony formation capacity when cells were treated with 100 µM of the connexins channels inhibitor carbenoxolone (CBX) for 7 days. The images show the proliferation and colony formation capacity of control cells (EV) and cells overexpressing Cx43 (Cx43) (n=3. Cx43 p value = 0.2820). Untreated cells (UT). These results indicate that the anti-tumour effects of Cx43 do not depend on the channel activity (hemichannels or gap junctions). **(e)** Cx43 re-expression in the A375 tumour cell line increases cellular senescence, detected by β-Galactosidase activity (n=4) and by flow cytometry (left, n =5), as well as by an increase in the senescence biomarkers p53 (n=2) and p16 (n=1) measured by western-blot (right). Ponceau staining was used as loading control. **(f)** The presence of Cx43 protein, increases tumour cell death by apoptosis measured by flow cytometry and double staining of PI/YO-PRO-1 (n=3). (**g**) The transfection of the human melanoma cell line Sk-Mel-147 (NRAS mutation) with a vector to restore Cx43 gene expression, leads to increased levels of Cx43 protein detected by western-blot (left, n=1). The presence of Cx43 significantly decreases the capacity to form colonies in Sk-Mel147 melanoma tumour cells. (**h**) In addition to melanoma, different tumour types with BRAF mutation, including breast cancer (MDA-MB-231 cell line) and colorectal cancer (HT29 cell line), were transfected with a EV and Cx43, and the levels of protein were detected by western blot (left, n=1). The presence of Cx43 significantly decreases the capacity to form colonies in both HT29 (n=3) and MDA-MB-231 (n=5) cells when transfected with a vector to overexpress Cx43.
   Data are presented as mean ± SEM. Mann-Whitney or Two-tailed Student's t-test were used to calculate the significance and represented as follows: * p<0.05, ** p<0.01, **** p<0.0001.

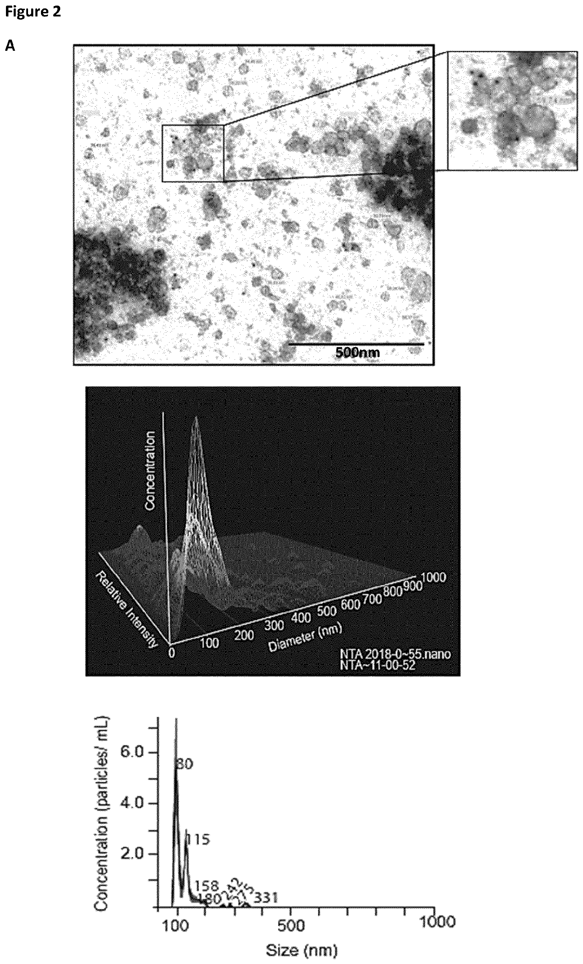

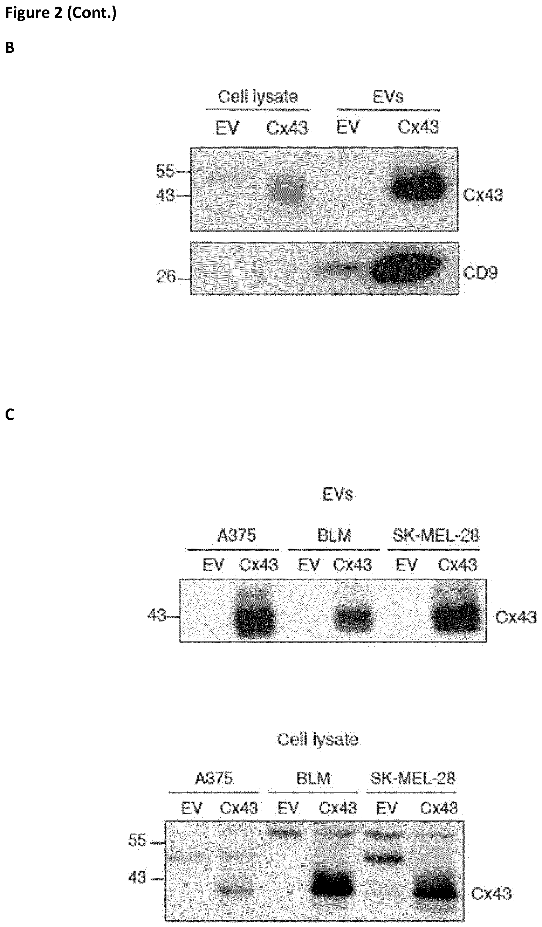

**Figure 2****. Cx43 levels are found in extracellular vesicles (EVs), including exosomes. (a)** EVs were isolated from A375 tumour cell line by ultracentrifugation and characterized by scanning electron microscopy (SEM). Representative images of the SEM analysis are shown on the left. Amplification of the image shows the size of the vesicles. Nanoparticle Tracking Analysis (NTA) shows the quantification and distribution of the particles, which range in size between 0 nm - 200 nm (size mean: 104.6 nm +/- 3.8 S.D. Concentration: 1.58e + 11 +/-1.69e + 09 particles/ml (n=3)). **(b)** Western-blot assays show Cx43 protein levels in melanoma tumour cell lysates (A375, right) and in EVs derived from control tumour cells transfected with the empty vector (EV) or with a vector to overexpress Cx43 (Cx43). CD9 were used to confirm the success of exosome isolation (n=1). **(c)** The analysis by western-blot shows Cx43 levels in whole cell lysate and EVs derived from different melanoma cells lines (A375, BLM, SK-MEL-28) transfected with the empty vector (EV) or with the vector to re-express Cx43 (Cx43).
**Figure 3****. The presence of Cx43 in EVs changes the protein and small RNA content. (a)** Proteomic analysis of extracellular vesicles (EVs) isolated from A375 melanoma cell line (Cx43 -) and A375 cells transfected with the Cx43 vector (Cx43 +). Data were analyzed considering false discovery rate (FDR) ≥0.82 for EVs Cx43 - and FDR ≥0.79 for EVs Cx43+ (n=4). Venn diagram shows the number of common proteins for both groups (1141), proteins only detected in EVs Cx43 - (160) and proteins only detected in EVs positive for Cx43 (482). **(b)** Proteomaps (*Liebermeister W. et al. 2014*) illustrates the KEGG Orthology and the biological process associated with low and high abundance of proteins observed in EVs Cx43 - and from EVs Cx43 +. In these Voronoi diagrams, each individual polygon represents one protein, wherein polygon area is a function of mass abundance. Each polygon is color-coded according to the KEGG Orthology term associated with it, and polygons representing the same KEGG Orthology term are clustered into larger polygons to form the map. **(c)** Gene set enrichment analysis (GSEA), Gene Ontology (GO), KEGG Orthology and Reactome data base were used to identify pathways and biological processes significantly enriched in EVs isolated from A375 tumour cells (Cx43 -, orange) and A375 overexpressing Cx43 (Cx43 +, blue). The analysis was performed only with proteins exclusively found in each group. (n=4); p-values are shown on the right. **(d)** Scatter plot illustrating the relationship between variance and correlation for the log10 mean and log10 standard deviation of the all proteins identified in both groups (Cx43 - and Cx43 +). Red circles represent proteins identified in Cx43 -, and blue squares are equivalent to proteins identified in Cx43 + (n=4). **(e)** RNA sequencing data of EVs Cx43 - and from EVs Cx43 +. Data were analyzed considering p-value<0.05 and fold change < -3,8 and > 3.8 (n=3). MiRNet 2.0 (Xia Lab, McGill) was used to identify pathways and biological processes enriched in Cx43 - (blue) and Cx43 + EVs (green). Pathways were selected based on enriched target genes of small RNAs mapped in both groups. P-values are shown on the right. **(f)** The data show the percentage and distribution of sequencing reads mapping to small RNA content in EVs negative for Cx43 and EVs positive for Cx43. The analysis was performed using GraphPad Prism 7.0. **(g)** Heatmap generated from RNA-seq data showing expression of miRNAs in Cx43 - and Cx43 + EVs. Some of the miRNAs found upregulated in Cx43 + EVs compared with CX43 - EVs are involved in process such as negative regulation of cell proliferation (hsa-mir-30c-2, hsa-mir-24-2), migration (hsa-mir-30c-2, hsa-mir-34a-5p, hsa-mir-24-2) and angiogenesis, (hsa-mir-34a-5p, hsa-mir-125b-5p) and positive regulation of cell death (hsa-mir-34a-5p). **(h)** Image shows the relative abundances of transcripts and fold changes between Cx43 - and Cx43 + EVs. The image represents the differential gene expression in both groups: green bars represent overexpressed genes in Cx43 + and red bars represent overexpressed genes in Cx43 -. Peptide identifications were accepted if they could be established at greater than 95,0% probability by the Scaffold Local FDR algorithm. Protein identifications were accepted if they could be established at greater than 99,9 % probability and contained at least 2 identified peptides. Protein probabilities were assigned by the Protein Prophet algorithm (Nesvizhskii, Al et al Anal. Chem. 2003;75(17):4646-58).

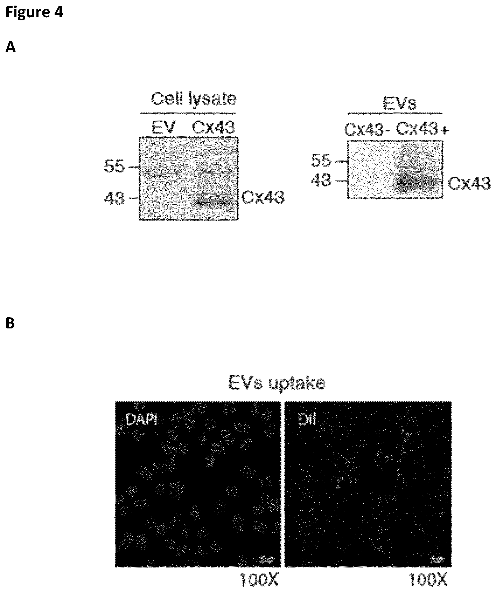

**Figure 4****. The presence of Cx43 in EVs switches the role of these vesicles promoting anti-tumour activity. (a)** Western-blot analysis to study the levels of Cx43 in EVs derived from A375 (Cx43 -) and from A375 overexpressing Cx43 (Cx43 +) show high levels of Cx43 in cell lysate (top panel) and EVs (low panel) of A375 when tumour cells overexpress Cx43 (Cx43) (n=1). **(b)** Purified EVs (isolated from cell culture media) were labeled with Dil (lipophilic membrane stain), to track its uptake by cells. DAPI was used as a nuclear stain. **(c)** Colony formation assay shows a reduction in cellular proliferation of A375 and BLM tumour cell lines after 7 days treatment with EVs containing high levels of Cx43 (Cx43 +) or without (Cx43 -). Quantification is shown on the right (A375, n=1 and BLM, n=1). **(c)** Western-blot to detect Cx43 protein in A375 cell line untreated (UT) in comparison with cells overexpressing Cx43 (A375-Cx43) and A375 cells treated with conditional medium for 48 hours (h) containing EVs derived from A375 cells overexpressing Cx43 (Cx43+EVs). Tubulin was used as loading control. Quantification is show on the right (n= 1). The effect of the conditional medium in Cx43 levels in A375 cells was confirmed by RT-qPCR. mRNA levels of Cx43 in A375 melanoma cells treated with EVs from A375 (Cx43 -) and with EVs from A375 overexpressing Cx43 (Cx43 +) is shown (n=1). Two-tailed Student's t-test were used to calculate the significance represented as follows: *** p <0.001, **** p < 0.0001

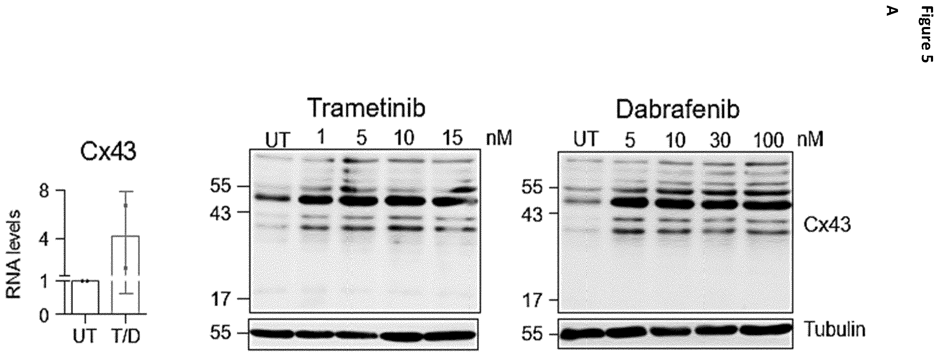

**Figure 5****. Cx43 levels increase when tumour cells are treated with BRAF/MEK inhibitors (a)** Western-blot showing Cx43 protein levels in A375 tumour cells treated with different concentrations of the BRAF inhibitor dabrafenib and MEK inhibitor trametinib for 72 h. Tubulin was used as loading control. Quantification by RT-qPCR (gene expression) is shown on the right: RT-qPCR to detect the increase of mRNA of Cx43 when tumour cells were treated with 0,5 nM of trametinib and 100 nM dabrafenib, for 72 h. Data are normalized to the untreated (n= 2). **(b)** The presence of Cx43 significantly decreases the capacity to form colonies in A375 melanoma tumour cells when the cells were transfected with a vector to overexpress Cx43 and were treated with different concentrations of dabrafenib and trametinib for 7 days. Colony formation assay quantification is shown on the right (n=1). **(c)** Flow cytometry analysis to detect SA-β-Galactosidase (FDG compound) activity in A375 containing the empty vector (EV) or the vector to overexpress Cx43 (Cx43) and treated with 100 nM dabra and 1 nM trame (100D/1T) for 5 days. Apoptosis was measured by double staining PI/YO-PRO-1 and analyzed using flow cytometry (n=3). **(d)** A375- EV and A375-Cx43 were treated with 3,5 µM NAV (navitoclax) for 24 h or with 1/100 nM of Trametinib/Dabrafenib (T/D) for 5 days. Cells were also treated with the combination of T/D for 4 days following by 24 h treatment with 3,5 µM NAV. Cleaved caspase 3 to detect apoptosis was studied by western-blot. Tubulin was used as loading control. Data are presented as mean ± SEM. Two-tailed Student's t-test was used to calculate the significance represented as follows: * p<0.05, ** p<0.01.

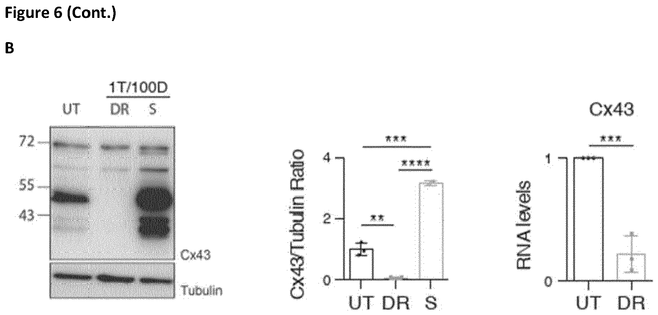

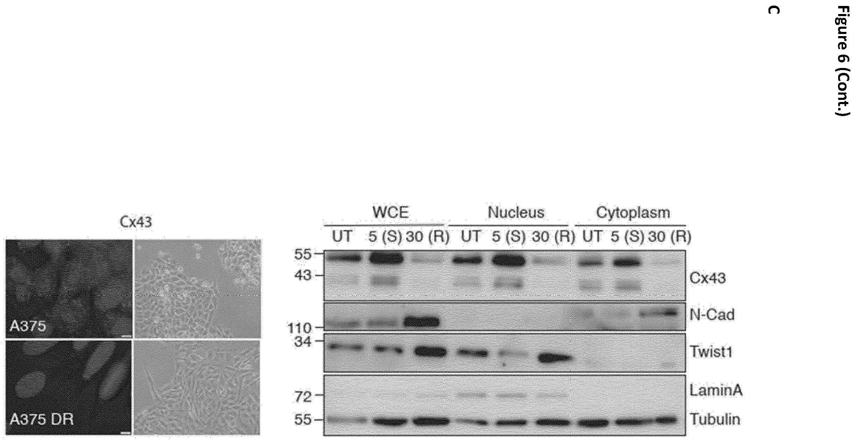

**Figure 6****. Resistant A375 tumour cells to BRAF/MEKi loss Cx43 and acquire a mesenchymal-like phenotype. (a)** Colony formation assay demonstrating that A375 resistant cells (DR) hardly respond to BRAF/MEKi compared to A375 control. Graphs on the right show the quantification (n=3). To obtain tumour cells resistant to BRAF/MEKi, cells were treated for 1 to 4 months with 100 nM and 1 nM of dabrafenib and trametinib. **(b)** Cx43 protein levels in A375 untreated cells and A375 resistant cells (DR) after 2 months exposed to 100 nM Dabra and 1 nM Trame compared to A375 cells sensitive cells to BRAF/MEKi (S) treated with 100D/1T for 72 h. Quantification is shown on the right (n = 3). Tubulin was used as a loading control. Cx43 gene expression was analyzed by RT-qPCR (right). Quantification of RNA levels (n= 3). Below: immunofluorescence showing Cx43 protein in A375 sensitive and resistant cells to BRAF/MEKi shows changes in cellular phenotype between sensible and resistant cells. **(c)** Whole cell extract, and nuclear and cytoplasm protein extract was analyzed by western-blot in untreated and A375 cells treated with 5 nM dabrafenib for 72 h (S) and during 2 months, in the presence of 20 nM dabrafenib (R). Epithelial-to-mesenchymal (EMT) markers indicate that when cells were sensible to the treatments (5 µM), the increase of Cx43 correlates with lower levels of nuclear Twist-1 and cytoplasmic N-cadherin, while when cells become resistant, after 2 months in culture, there is an increase in nuclear Twist-1 and cytoplasmic N-cad correlating with loss of Cx43 protein. Tubulin and nuclear Lamin A were used as a loading controls. **(d)** A375 cells overexpressing Cx43 after transfection with a vector (Cx43) show significantly less cell proliferation and colony formation capacity after 1 - 2 weeks and 4 months under treatment with BRAF/MEKi (1 nM T/ 100 nM D). The presence of Cx43 increases the efficacy of the therapy and avoids the acquisition of drug resistance (n = 3). Almost no cells were detected after 2 weeks of treatments in the presence of Cx43. **(e)** Restoration of Cx43 levels in resistant A375 tumour cells (DR) by using a vector sensitizes tumour cells to cell death by apoptosis. Apoptosis was measured by double staining of PI/YO-PRO-1 and analyzed by flow cytometry. Quantification is shown on the right. Cellular phenotype is shown on the left (n =1). Data are presented as mean ± SEM. Two-tailed Student's t-test or Mann- Whitney test was used to calculate the significance represented as followed: * p<0.05, ** p<0.01 and ***p<0.001.
**Figure 7****. EVs containing high levels of Cx43 have anti-tumour activity and improve BRAF/MEK inhibitor efficacy. (a)** Western-blot showing Cx43 levels in A375 melanoma cells and in EVs derived from A375 cells under treatment with BRAF/MEKi (0,5 nM trame and 30 nM dabra) for 72 h. Tubulin (Tub) and CD9 were used as loading controls. Quantifications are shown on the right (n = 1). **(b)** Western-blot from cell lysate and EVs derived from HEK293 cells. CD9 and CD63 were used as loading controls (n = 2). EVs from HEK293 contain high levels of Cx43. **(c)** Colony formation assay of A375 melanoma cells treated with 30 nM dabra and 0.5 nM trame and EVs-Cx43 + isolated from HEK293 for 5 days (n = 4). The presence of EVs enriched in Cx43 significantly decreases the capacity to form colonies and increases the efficacy of treatment. Treatments and EVs were refreshed every 48 h. UT (untreated), Cx43 + (EVs derived from HEK293). **(d)** Tumour resistant A375 cells to BRAF/MEKi were exposed to 2 or 0.5 nM trame and 200 or 30 nM dabra, in the presence or absence of EVs enriched in Cx43 isolated from HEK293 (Cx43 +). The treatment of melanoma resistant cells to BRAF/MEKi with EVs Cx43 + has no significant effect on colony formation (left), while the combination of EVs Cx43 + with both inhibitors re-sensitizes the resistant cells to the treatment. Colony formation assay was performed for 5 days and treatments were refreshing every 48 h (n = 4). **(e)** Colony formation assay of A375 melanoma cell line treated with the senolytic compound NAV (ABT-263, 3,5 µM) and Cx43 + EVs for 5 days. Treatments were refreshed every 48 h. Quantification is shown on the right (n = 4). **(f)** A375 melanoma resistant cells to BRAF/MEKi were treated with 0,5 µM NAV in combination with 2 nM trametinib and 200 nM dabrafenib, in the presence and absence of EVs positive for Cx43 for 5 days. Treatments were refreshed every 48 h. Quantification are shown on the right (n = 4). Data are presented as mean ± SEM. Two-tailed Student's t-test and one-way ANOVA were used to calculate the significance represented as follows: * p<0.05, ** p<0.0, ***p<0.001.

### Detailed description of the invention

The present invention is illustrated by means of the examples set below without the intention of limiting its scope of protection.

### Example 1. Materials and Methods.

### Example 1.1. Cell Culture.

The BRAF mutated tumour cell lines A375 (melanoma), SK-Mel-28 (melanoma), MDA-MB-231 (human breast cancer) and HT-29 (human colorectal adenocarcinoma) were also used to test the role of Cx43 in drug resistance. SK-Mel147 (melanoma), NRAS mutated cell line was selected to compare the results with BRAF mutant cell lines. Cells were cultured in Dulbecco's modified Eagle's medium (DMEM) (Lonza) supplemented with 10 % fetal bovine serum (FBS) (Gibco, Thermo Fisher Scientific) and 100 U/mL penicillin and 100 µg/ml streptomycin (Gibco, Thermo Fisher Scientific). HT-29 were cultured in McCoy'S 5A medium (Sigma-Aldrich) supplemented with 10 % fetal bovine serum (FBS) (Gibco, Thermo Fisher Scientific) and 100 U/mL penicillin and 100 µg/ml streptomycin (Gibco, Thermo Fisher Scientific). The cells lines were maintained in sterile conditions under a flow laminar hood (Telstar BIO II A). Cells were maintained at 37 °C in 5 % CO₂ humidified incubator (SANYO CO²). The medium was changed every 2-3 days. To detach the cells, the medium was removed, and cells were washed with a saline solution (Fresenius Kabi). Trypsin (Gibco, Thermo Fisher Scientific) was added to the dish and incubated at 37 °C for several minutes (min). After adding DMEM supplemented with 10 % FBS, cells were transferred to falcon tube and counted using the Neubauer chamber method in a light microscope. Cells were centrifuged at 1800 revolutions per minute (rpm) for 5 min at room temperature (RT) before seeding in new plates to use them for experimental analysis or to treat them with different drugs. The BRAF inhibitor Dabrafenib (D) (Tafinlar^{®} 75 mg, Novartis) was dissolved in dimethyl sulfoxide (DMSO) at 1mM final concentration and stored at -20 °C. The MEK inhibitor Trametinib (T) (Mekinist^{®} 2 mg, Novartis) was dissolved in DMSO (1mM) and stored at -20 °C. To obtain double resistance cells (DR) to T and D, tumour cells were treated for 1 to 6 months under the presence of both inhibitors (started with a low dose (0.5 nM T + 30 nM D) and escalated as and when cell lines were confluent (2 nM T + 200 nM D). Concentrations (initial and maintenance) used were depended on the sensibility of the cell lines to the treatments. Carbenoloxolone disodium salt (CBX) (Sigma-Aldrich) was dissolved in distilled water (dH₂O) at 20 mM final concentration and stored at -4 °C.

### Example 1.2. Cell Transfection.

Cell lines were transfected by electroporation. The Amaxa^{®} Cell Line Nucleofector^{®} Kit V (Lonza) was used to transfect cells in a Cell Line Nucleofector^{™} Device (Lonza). A million cells were harvested and resuspended in 100 µL of Nucleofector^{®} solution. 3 µg of the corresponding plasmid was added, and the mixture was transferred into an electroporation cuvette using the U-28 program. Cell lines were transfected with pIRESpuro2 plasmid construct (Clontech) containing the human Cx43 sequence, kindly donated by Arantxa Tabernero (Institute of Neuroscience of Castilla y León, University of Salamanca, Spain). The transfected cells were seeded in culture medium and 24 hours (h) post-transfection the medium was replaced with complete medium containing puromycin dihydrochloride (Tocris, Bioscience) at different concentrations depending of the cell line (0,5 µg/mL to 2 µg/mL).

### Example 1.3. Western blot.

Cells were harvested and washed twice with saline before protein isolation. Total cell lysates were obtained by disaggregating the cells with an insulin 30-gauge syringe (Omnican, Braun) in ice- cold lysis buffer composed by 150 mM NaCl, 50 mM Tris-HCL (pH 7.5), 5 mM EDTA (pH 8), 0.5 % (v/v) Nonidet P-40, 0.1 % (w/v) Sodium Dodecyl Sulfate (SDS), 0.5 % (v/v) Sarkosyl (all from Merck) supplemented with 0.1 mM phenylmethysulfonyl fluoride (PMSF) and 1X Protease Inhibitors Cocktail (Sigma-Aldrich. Loading buffer (10 % (v/v) β-mercaptoethanol (Merk); 10 % SDS, 50 % glycerol (v/v); 200 mM Tris-HCl pH 6.8, 0.1 % bromophenol blue) was added to protein extracts and boiled at 99 °C for 10 min. Protein concentrations were measured using a Nanodrop^{®} ND-1000 (Thermo Fisher Scientific) and 30 µg of protein samples were used to perform the SDS-PAGE electrophoresis in 10 % or 15 % AcrylamideBis-acrylamide gels and subsequently transferred to polyvinylidene fluoride (PVDF) membranes (Inmobilon-P, Millipore) using Mini Trans-Blot Cell System (Bio-Rad Laboratories). Transfer was performed at 100 V during 1 or 2 h in ice cold. After transference, membranes were stained with ATX Ponceau S red staining solution (Merk) for 15 min at RT and then unstained with several washes with distilled water. Membranes were blocked with 5 % skin milk (Merck) in tris-buffered saline with 0.05 % Tween-20 (TBS-T) for 1 h at RT. Primary antibodies and secondary antibodies were diluted in 5 % skin milk TBS-T incubated overnight (O/N) at 4 °C or 1 h at RT, respectively. HRP-conjugated secondary antibodies used were anti-mouse (NA-931, Sigma-Aldrich) and anti-rabbit (A6154, Sigma-Aldrich). Signal was developed using PierceTM ECL Western Blotting Substrate (Thermo Fisher Scientific) in either a LAS-3000 Imager (Fujifilm) or an Amersham Imager 600 (GE Healthcare). Image J software was used to quantify protein band intensities.

For nuclear protein isolation the NE-PER^{™} Nuclear and Cytoplasmic Extraction kit (Thermo Fisher Scientific) was used following manufacture's protocol. For membrane (insoluble) /cytosol (soluble) protein, pelleted cells were lysed in 1 % Triton X-100 (v/v) in phosphate-buffered saline (PBS: MP Biomedicals) supplemented with 0.1 PMSF and 1X Protease Inhibitors Cocktail. The cells were intermittently vortexed and kept on ice for 1 h. Lysates were centrifuged at 10,000 g for 15 min at 4 °C and supernatant was collected (cytosolic soluble proteins). The insoluble fraction was resuspended in lysis buffer (membrane). The primary antibodies used were: anti- Cx43 (#C6219), Sigma- Aldrich 1: 1000; anti-Tubulin (T9026), Sigma- Aldrich 1: 5000; anti-N-cadherin (#13116), Cell Signaling 1: 1000; anti-CDKN2A/p16INK4a (ab108349), Abcam 1: 1000; anti-p53 (Sc-126, Santa Cruz) 1: 500; anti-p44/42 MAPK (Erk1/2) (#9102), Cell Signaling 1: 1000; anti-Twist-1 (Sc-81417), Santa Cruz 1:100; anti-Lamin A (Sc-20680), Santa Cruz 1: 1000; anti-CD9 (Sc-9148), Santa Cruz 1: 1000; anti-CD63 (Sc-15363), Santa Cruz 1: 1000. The secondary antibodies used were: Goat anti-Rabbit (A6154), Sigma-Aldrich 1: 1000 to 1: 5000; Sheep anti- Mouse (NA-931), Sigma-Aldrich 1: 1000 to 1: 10000.

### Example 1.4. Immunofluorescence on fixed cells.

1×10⁴ cells were cultured on coverslips (Thermo Fisher Scientific) until 80 - 90 % confluence, washed with PBS and fixed with 2% (w/v) paraformaldehyde (PFA: Sigma-Aldrich) in PBS for 15 min at RT. The cells were incubated twice for 10 min each with 0.1 M glycine (Sigma-Aldrich), permeabilized with 0,2 % Triton X-100 (Sigma-Aldrich) in PBS for 10 min, washed twice with PBS and incubated with 1 % bovine serum albumin (BSA; Sigma-Aldrich) at RT for 30 min. Fixed cells were incubated with primary antibody diluted in 1 % BSA in PBS supplemented with 0.1 % (v/v) Tween 10 (PBS-T) O/N at 4 °C. Cells were then washed three times with PBS and incubated with Alexa-conjugated secondary antibodies (diluted in 1 % BSA in PBS-T) at RT for 1 h in the dark. Cells were washed three times with PBS and counterstaining of nuclei with 1 µg/mL of 4',6-diamidino-2-phenylindole dihydrochloride (DAPI; Sigma-Aldrich) for 5 min at RT in the dark and rinsed three times with PBS. Mounting the cover slips were done with a drop of glycergel aqueous mounting medium (Dako) on a glass microscope slide. Negative controls were obtained by omitting the primary antibody. The primary antibody used were: anti- Cx43 (#C6219), Sigma-Aldrich 1: 500; The secondary antibody used were: Goat anti-Rabbit (F-2765), Thermo Fisher Scientific 1: 100.

### Example 1.5. Scrape Loading assay.

Cells were seeded on 6 or 12 wells and cultured until 80 - 100 % of confluence and rinsed twice with warm PBS. Lucifer Yellow CH dilithium salt (1 mg/mL) (LY: Cell Projects Ltd^{©} Kent) in PBS was added to the cells and parallel cuts (3 cuts per well) were performed with a surgical blade (Swann-Mortonscalpel^{®}). After 3 - 5 min at 37 °C cells were washed with PBS and fixed 2 % PFA in PBS. LY becomes incorporated by cells along the cut and move from the dye-loaded cells into adjacent ones connected by functional gap junction channels. The areas for LY loading were randomly selected in the central part of wells to obtain the images for quantification. Images were captured with 10x objective using a Nikon Eclipse Ti fluorescent microscope. The constant exposures were used for all images for a given experiment. Ten images per sample were quantified. The score was calculated as the ratio of the number of non-damage LY positive cells at the edge and the number of the LY positive cells outside the edge.

### Example 1.6. Dye Coupling.

Cells were seeded on 12-wells plated and cultured until 80-100 % of confluence. Glass micropipettes were backfilled with 133 mM of 5- or 6-carboxyfluorescein with a negative charge (-2) (Sigma-Aldrich) diluted in potassium acetate 0.1 M (Sigma-Aldrich). One cell was injected and hyperpolarizing pulses were applied with an intensity of 0.25-1 nA. Cells were excited with 488 nm in a confocal microscopy and the image was registered in Lasershap (MRC-1024, BioRad). In the image acquisition process, regions of interest (or ROI) were defined to detect the cell that was injected, the donor cells and the adjacent receptor cells. Before injecting the cell, the gain of the photomultipliers was reduced below 20 % of the dynamic detection range (from 0-250), which was taken as the basal value. The fluorescence emitted was measured at 640/40 nm and 540/40 nm with two photodetectors, the time course of the passage of dye from the injected cell to the adjacent cells was studied.

### Example 1.7. RNA isolation and reverse transcription polymerase chain reaction (RT-qPCR).

Total RNA was isolated from cells using TRI Reagent^{®} RT (Vitro Bio) according to manufacturer's protocol. 5×10⁵ to 10⁶ cells were harvested and lysed in 1 mL TRIzol reagent. 230 µL of chloroform were added to the tubes and they were vortexed during 1 min. Samples were incubated for 10 min at 4 °C and then centrifugated at 14,000 g for 15 min at 4 °C. After centrifugation, the aqueous phase (upper) are collected and transferred to a new tube with 500 µL of isopropanol and samples were vortexed 15 sec. The tubes were incubated for 30 min at -20 °C followed by 14,000 g centrifugation for 15 min at 4 °C. Supernatant was discarded, and the RNA pellets were washed with 1 mL of 70 % ethanol and centrifugated for 5 min at 7,000 g at 4 °C. The supernatants were discarded, and RNA pellets were air dry for 2 h at 4 °C. RNA was treated with DNase I, RNase free (Thermo Fisher Scientific) following manufacturer's protocol. RNA samples were quantified in a Nanodrop^{®} ND-1000 (Thermo Fisher Scientific). 1 µg of RNA was used to synthesize complementary DNA (cDNA) with the Superscript^{™} IV VILO^{™} Master Mix (Invitrogen, Thermo Fisher Scientific). Samples were denatured for 10 min at 65 °C, and 2 µL of the master mix were added. Samples were incubated using a thermo cycler (Veriti, Applied Biosystems) for 10 min at 25 °C, followed by 60 min at 42 °C and 5 min at 85 °C. Finally, cDNA samples are quantified in a Nanodrop^{®} ND-1000 (Thermo Fisher Scientific) and resuspended in DNase/RNase/Protease-free water (Sigma-Aldrich).

### Example 1.8. Real time quantitative PCR (RT-qPCR).

5 µL of cDNA (1µg) were mixed with 0.5 µL of primer mix (10 µM each primer), 10 µL of Applied Biosystems^{™} PowerUP^{™} SYBR^{™} Green Master Mix (Applied Biosystems, Thermo Fisher Scientific) and complete with dH₂O until 20 µL per well on LightCycler^{®} 480 System (Roche). The program consisted on a first denaturing cycle of 10 min at 95 °C followed by 30 - 55 amplification cycles for 10 seconds (sec) at 95 °C, 30 sec at 60 °C for annealing and 12 sec at 72 °C for extension. The primers used were: GJA1 (Cx43): Forward: SEQ ID NO: 1; Reverse: SEQ ID NO: 2. HPRT1: Forward: SEQ ID NO: 3; Reverse: SEQ ID NO: 4.

### Example 1.9. Cell Counting Kit-8 (CCK-8).

Dojindo's highly water-soluble tetrazolium salt (WST-8) is reduced by dehydrogenase activity in cells to give a yellow-color formazan dye, which is soluble in the tissue culture media. The amount of the formazan dye generated by dehydrogenases is directly proportional to the number of living cells. Equal number of cells (1000 - 2000 cells) were seeded per triplicate in 96-well plates and cultured in the different conditions. Medium was replaced by 100 µL of fresh medium and 10 µL of CCK-8 reagent was added per well. After an incubation for 1 to 4 h at 37 °C, absorbance was measured at 450 nm in a NanoQuant microplate reader Infinite M200 (TECAN). Results were calculated as optical density (OD) 450 nm = OD of sample - OD of blank.

### Example 1.10. xCelligence System.

The E8 xCELLigence plates were prepared by addition of 100 µL of complete media to every well. After incubation at 37 °C, plates were inserted into the xCELLigence station, and the base-line impedance was measured to ensure that all wells and connections were working. Following harvesting and counting, cells were diluted to the selected seeding density and 100 µl of cells in culture medium was added to each well. For the cell proliferation assays for each condition, 1 × 10³ - 5 × 10³ cells / well was seeded into 100 µL of media in the E-Plate L8 (Acea Biosciences). The attachment and proliferation of the cells were monitored every 30 min. Cell proliferation was monitored for 48 - 72 h. Cell Index at each time point is defined as (Rₙ-R_{b})/15, where Rₙ is the cell-electrode impedance of the well when it contains cells and R_{b} is the background impedance of the well with the media alone. For the cell adhesion assays, for each condition, 1 × 10³ - 5 × 10³ cells / well was seeded into 100 µL of media in E-Plate L8 (Acea Biosciences). Cell proliferation was monitored for 1 h every 10 sec. The extend of cell adhesion and spreading was monitored every min for 1 - 3 h. Cell Index at each time point is defined as (Rₙ-R_{b})/15, where Rₙ is the cell-electrode impedance of the well when it contains cells and R_{b} is the background impedance of the well with the media alone.

### Example 1.11. Click-iT Assay.

Cells proliferation by tracking new DNA synthesis was performed with Alexa Fluor 647^{™} Click-iT^{®} Assays kit (Thermo Fisher Scientific), following manufacturer's instructions. Briefly, cells were previously cultured in a serum-free medium and incubated with 10 µM of 5-ethynyl-2'-deoxyuridine (EdU), a nucleoside analog to thymidine, for 2 h to be incorporated into DNA during S phase (DNA synthesis). Cells were harvested, fixed, and incubated with the Alexa 647^{™} picolyl azide. EdU incorporation was analyses by flow cytometry using a FACs Scalibur and acquiring 20,000 event per triplicate. Data was analyzed using the FCS Express 6 Flow software.

### Example 1.12. Clonogenic Assay.

Colony formation assays were performed by seeding 5 × 10³ to 5 × 10⁴ cells per well onto 6 - 12 well plates and grown for 7 - 15 days. The culture medium was replaced every 48 h. The cells were washed with warm Saline Solution and fixed with cold 2 % PFA for 15 min. Cells were washed with 1 x PBS and stained with 0.1 % of crystal violet (Sigma-Aldrich) for 15 min. After staining, the cells were washed with distilled water and dried at room temperature. The colonies were quantified by diluting crystal violet in 5 % acetic acid and 100 µL of the solution was collected to measure the absorbance at 570 nm using a NanoQuant microplate reader Infinite M200 (TECAN).

### Example 1.13. Cellular senescence assay.

The detection of SA-β-Gal activity was measured using the Senescence Cells Histochemical Staining Kit (Sigma-Aldrich). Cells were rinsed with warm PBS and fixed for 7 min at RT with a fixation buffer composed by 2 % paraformaldehyde and 0.2 % glutaraldehyde. After three washed with PBS, cells were incubated for 6 h or O/N at 37 °C without CO₂ in a staining solution containing X-gal. This lactose analogue can be cleaved by β-galactosidase producing a blue insoluble product. Finally, cells were washed with PBS and images were taken using a light microscope.

### Example 1.14. SA-β-Gal detection by flow cytometry.

SA-β-Gal activity was analyzed with the fluorogenic β-Galactosidase substrate di-β-D-galactopyranoside (FDG, Thermo Fisher Scientific), which is hydrolyzed by the endogenous β-galactosidase to fluorescein (FITC). Cells were harvested and incubated in a staining medium (PBS, 4 % FBS, 10 mM HEPES, pH 7.2) at 37 °C for 10 min. The β-Galactosidase assay was started by adding warm 2 mM FDG to the cell suspension and after incubating for 3 min at 37 °C in the dark. The reaction was stopped with the addiction of ice-cold staining medium to the cells, which were kept on ice protected from light. 515 nm laser was used for fluorescein and acquiring 20,000 event per triplicate. Data was analyzed using the FCS Express 6 Flow software.

### Example 1.15. Apoptosis assay.

Supernatants (died cells) were aspirated and attached cells were harvested. Supernatants and cells were mixed, counted and 1 million cells were resuspended in 1 mL in flow cytometry (FC) buffer. Membrane viability analysis was performed by double staining PVYO-PRO-1. YO-PRO-1 (Invitrogen) was added to the cells (150 nM) and incubated 10 min in the dark at 4 °C. PI (Invitrogen) was added (2 µg/mL) and incubated for 5 min in the dark at 4 °C. After the incubation time (PI/YO-PRO) samples were analyzed in a FACs Scalibur acquiring 20,000 event per triplicate. The red fluorescence emitted by PI was detected at 610 nm and the green florescence emitted by YO-PRO was detected at 515 nm. Data was analyzed using the FCS Express 6 Flow software.

### Example 1.16. Extracellular vesicles (EVs) purification.

Cells were seeded into 162 cm² culture flasks (Corning, Sigma-Aldrich) until confluence (80 % - 90 %). Cells were washed three times with PBS and cultured for 48 h in DMEM 0 % FBS. Supernatants were collected and centrifuged at 1800 rpm for 5 min. Pellet was discarded and supernatants were filtered through a 0.22 µm filter and then, the filtered supernatants, were centrifuged at 100,000 g for 90 min at 4 °C (Optimal-90K ultracentrifuge) using 70 Ti rotor (Beckman Coulter). Supernatants were discarded and pellet-containing EVs were washed with PBS and centrifuged at 100,000 g for 90 min at 4 °C. The pellet with EVs was resuspended in culture medium, PBS or lysis buffer, depending on the analysis performed. The EVs were characterized by scanning electron microscopy and by the NTA Nanosight NS300.

### Example 1.17. Immunogold and scanning electron microscopy.

Purified EVs were fixed in 2 % PFA, and 5 µL were deposited on formvar-carbon coated grids and let them air-dry for 1 h. Grids were then washed three times with PBS, followed by a 10 min incubation with 0,1 M glycine. Grids were incubated with 1 % BSA in PBS for 10 min at RT. Grids were fixed with 1 % glutaraldehyde for 5 min, followed by several washed with distilled water. Grids were stained in uranyl-oxalate pH 7 for 5 min and then contrasted in a mixture of 4 % uranyl acetate and 2 % methyl cellulose for 5 min on ice. Grids were dried and stored until visualize using JEOL JEM 1010 transmission electron microscope.

### Example 1.18. Labelling of EVs.

Isolated EVs were stained in 100 µL of PBS with 1 µM Dil for 1 h at 37 °C. After them, EVs were washed in 10 mL of PBS and centrifuged at 100,000 g for 90 min at 4 °C. The pellet was resuspended in culture medium for different experiments.

### Example 1.19. Proteomics.

EVs from tumour cell lines A375-EV and A375-Cx43 were collected in lysis buffer (150 mM NaCl, 50 mM Tris-HCl (pH 7.5), 5 mM EDTA (pH 8), 0.5 % (v/v) Nonidet P-40, 0.1% (w/v) SDS, 0.5 % (v/v) Sarkosyl) for protein isolation. In order to make global protein identification, an equal amount of protein (100 µg) from all conditions were loaded on a 10 % SDS-PAGE gel. The run was stopped when the front had penetrated 3 mm into the resolving gel. The protein band was visualized by Sypro-Ruby fluorescent staining (Lonza), excited, and subjected to in-gel, manual tryptic digestion following the protocol defined by Shevchenko (Shevchenko) with minor modifications. The peptides were extracted three times by incubation in 40 µL of a solution containing 60% ACN and 0.5% formic acid (HCOOH) for 20 min. The resulting peptide extracts were pooled, concentrated in a SpeedVac and stored at -20 °C ³. For protein identification by LC-MS/MS, 4 µl (aprox. 4 µg) of digested peptides of each sample were separated using Reverse Phase Chromatography. Gradient was developed using a micro liquid chromatography system (Eksigent Technologies nanoLC 400, SCIEX) coupled to high speed Triple TOF 6600 mass spectrometer (SCIEX) with a micro flow source. The analytical column used was a silica-based reversed phase column Chrom XP C18 150 × 0.30 mm, 3 mm particle size and 120 Å pore size (Eksigent, SCIEX). The trap column was a YMC-TRIART C18 (YMC Technologies) with a 3 mm particle size and 120 Å pore size, switched on-line with the analytical column. The loading pump delivered a solution of 0.1% formic acid in water at 10 µL/min. The micro-pump provided a flow-rate of 5 µL/min and was operated under gradient elution conditions, using 0.1 % formic acid in water as mobile phase A, and 0.1 % formic acid in acetonitrile as mobile phase B. Peptides were separated using a 90 min gradient ranging from 2 % to 90 % mobile phase B (mobile phase A: 2 % acetonitrile, 0.1 % formic acid; mobile phase B: 100 % acetonitrile, 0.1 % formic acid). Data acquisition was carried out in a TripleTOF 6600 System (SCIEX) using a data dependent workflow (DDA). Source and interface conditions were as follows: ionspray voltage floating (ISVF) 5500 V, curtain gas (CUR) 25, collision energy (CE) 10 and ion source gas 1 (GS1) 25. Instrument was operated with Analyst TF 1.7.1 software (SCIEX). Switching criteria was set to ions greater than mass to charge ratio (m/z) 350 and smaller than 1400 (m/z) with charge state of 2-5, mass tolerance 250 ppm and an abundance threshold of more than 200 counts (cps). Former target ions were excluded for 15 s. Instrument was automatically calibrated every 4 h using as external calibrant tryptic peptides from pepcalMix. The mass spectromic analysis were performed in the Proteomics Facility of the Instituto de Investigación Sanitaria de Santiago de Compostela (IDIS). After MS/MS analysis, data files were processed using ProteinPilot^{™} 5.01 software (SCIEX), which uses the algorithm Paragon^{™} for database search and Progroup^{™} for data grouping. Data were searched using a Human specific Uniprot database. False discovery rate (FDR) was performed using a non-lineal fitting method displaying only those results that reported a 1 % Global false discovery rate or better (shilov IV and Tang WH).

### Example 1.20. Proteomic data analysis.

Differential gene expression levels between the EVs derived from A375 control (EVs Cx43-) and EVs derived from A375 overexpressed Cx43 (EVs Cx43+) were estimated based on the false discovery rate (FDR). Proteomic analysis was performed considering FDR ≥0.82 for EVs Cx43- and FDR ≥0.79 for EVs Cx43+. All calculations were performed using GraphPad Prism 7.0. Proteomaps were constructed using a web tool based on the t-test difference values (Liebermeister et al., 2014). The enrichment analysis was done using GSEA software version 4.0.3 (Subramanian et al. 2005) (Gene Ontology (GO), KEGG Orthology and Reactome data base). For the present study, enrichment analysis was performed based on FDR of data set of each group of specific proteins (EVs Cx43- and EVs Cx43+). GSEA analysis heat maps are shown by Prism based on Normalized Enrichment Score (NES) and FDR. Scaffold (version Scaffold_4.11.0, Proteome Software Inc., Portland, OR) was used to validate MS/MS based peptide and protein identifications. Peptide identifications were accepted if they could be established at greater than 95,0% probability by the Scaffold Local FDR algorithm. Protein identifications were accepted if they could be established at greater than 99,9% probability and contained at least 2 identified peptides. Protein probabilities were assigned by the Protein Prophet algorithm (Nesvizhskii, Al et al Anal. Chem. 2003;75(17):4646-58). Proteins that contained similar peptides and could not be differentiated based on MS/MS analysis alone were grouped to satisfy the principles of parsimony. Proteins sharing significant peptide evidence were grouped into clusters.

### Example 1.21. RNA-seq.

Tumour cells A375-EV and A375-Cx43 were cultured in 175 cm² flasks until 80 % confluence. 10 mL of cell culture media was collected and centrifuged at 1500 x g for 5 min to remove cells and debris. The supernatant was transferred to a new 50 mL conical tube for exosome (EVs) isolation. ExoQuick-TC (System Biosciences) was added to the supernatant at 1:5 ratio (ExoQuick: Supernatant), mixed gently, and allowed to incubate O/N at 4 °C. Next day, the admixture was centrifugated at 1500 x g for 30 min to recover exosomes for total RNA isolation. The remaining exosomes from samples were processed for total RNA isolation using the SeraMir Exosome RNA Purification Column kit (System Biosciences) according to the manufacturer's instructions. For each sample, 1 µL of the final RNA eluate was used for measurement of small RNA concentration by Agilent Bioanalyzer Small RNA Assay using Bioanalyzer 2100 Expert instrument (Agilent Technologies). Small RNA libraries were constructed with the Clean Tag Small RNA Library Preparation Kit (TriLink) according to the manufacturer's protocol. The final purified library was quantified with High Sensitivity DNA Reagents (Agilent Technologies). The libraries were pooled, and the 140 bp to 300 bp region was size selected on an 8 % TBE gel (Invitrogen, Life Technologies). The size selected library is quantified with High Sensitivity DNA 1000 Screen Tape (Agilent Technologies). High Sensitivity D1000 reagents (Agilent Technologies), and the TailorMix HT1 qPCR assay (SeqMatic), followed by a NextSeq High Output single-end sequencing run at SR75 using NextSeq 500/550 High Output v2 kit (Illumina) according to the manufacturer's instructions. The Exosome Small RNA-seq Analysis Kit initiates with a data quality check of the input sequence using FastQC, an open- source quality control (QC) tool for high-throughput sequence data. FastQC runs analyses of the uploaded raw sequence reads that quality of the data and inform the subsequent preprocessing steps in the analysis. Following QC, the analysis moves to preprocessing of the RNA-seq reads to improve the quality of data input for read mapping. The open-source tools used are FastqMcf, part of the EA-utils package, and PRINSEQ. Data preprocessing detects and removes N's at the ends of reads, trims sequencing adapters, and filters reads for quality and length. FastQC is then rerun to analyze the trimmed reads, allowing a before and after comparison. The summary report generated provides a quality assurance check to validate the processed set of input data used in the subsequent read mapping step. The improved set of sequence reads are mapped to the reference genome using Bowtie, an ultrafast, memory-efficient short read aligner, followed by the generation of a mapping summary report for review. Using the open-source software SAMtools and Picard expression analyses are carried out determination of ncRNA abundance and differential expression analysis across samples. Expression statistics are calculated and visualized using R software for statistical computing and graphics. The Exosome Small RNA-seq Analysis Kit identifies and maps miRNAs, tRNAs, small rRNAs, repeat elements, antisense transcripts and a variety of small ncRNAs. The Exosome Small RNA-seq analysis produces a summarization of results, including expression statistics and chromosome distribution, as well as genome browser tracks of read alignment and read coverage for analysis in a genomic context.

### Example 1.22. RNA-seq data analysis.

Data were analyzed considering p-value <0.05 and fold change <-3,8 and >3.8. Genes were considered as differentially expressed if the comparison of relative abundance between both groups is positive or negative. The list of differentially expressed genes in EVs Cx43- and EVs Cx43+, containing gene identifiers and corresponding expression values (fold changes), were uploaded into the miRNet 2.0 (Xia Lab, McGill) online tool. The analysis included biological processes, canonical pathways, biological processes, molecular functions and gene networks. Graphics were performed using GraphPad Prism 7.0.

### Example 2. Results.

### Example 2.1. Restoration of Cx43 gene expression in cancer cells with a mutation in BRAF and NRAS has anti-tumour effects.

The primary melanoma cell line A375 were derived from human melanoma tissue. Decreased expression of connexins (Cxs) have been observed in melanoma cell lines. Cx43 expression was analysed in A375 human metastatic melanoma cell line. To address the direct effect of Cx43 in melanoma, Cx43-expressing vector (Cx43) or empty vector (EV) were transfected in A375 cells. Cx43 overexpression was confirmed by western blot, flow cytometry analysis, RT-qPCR and immunofluorescence (IF) staining **(****Figure 1a**). Overexpression of Cx43 was associated with the presence of Cx43 in the membrane forming the gap junction (GJ) plaque at the cell-cell interface (red arrows in IF, **Figure 1a**). In accordance with these results, Cx43 transfected cells showed increased levels of dye coupling compared with control EV (**Figure 1b**), demonstrating that the cells form functional GJ channels. These results were checked with two different techniques, by dye transfer and by scrape loading **(****Figure 1b**). Cx43 overexpression induces a significant reduction in the proliferation rate of A375 cells with a mutation in BRAF **(****Figure 1c**). These results were analysed by xCelligence technology, colony formation assay and CCK8 assay. Cx43 has channel-dependent (hemichannels and GJ) and channel-independent functions. To know whether the blockade of channels leads to significant reduction in proliferation ratio, we used the channel blocking agent carbenoxolone (CBX) to inhibit Cx43 channels functions. When we treated the cells (EV and Cx43) with CBX, we have not detected changes in cellular proliferation when Cx43 was overexpressed (**Figure 1d**). This finding indicates that the role Cx43 in cellular proliferation is channel-independent. Cellular senescence is broadly defined as the physiological program of terminal growth arrest. Cells that undergo senescence cannot divide even if stimulated by mitogens, but they remain metabolically and synthetically active and show characteristic changes in morphology. The most widely used marker to senescence cells is SA-β-gal, which was detectable by flow cytometry and staining in EV and Cx43 cells (**Figure 1e**). In **Figure 1e****,** we have confirmed by western blotting that Cx43 cells expressed higher levels of the senescent biomarkers p53 and p16 than control cells (transfected with the EV). To determine whether Cx43 is involved in the control of cell survival in human melanoma cells, we measured percentage of apoptotic cells when Cx43 was overexpressed. The basal percentage of early apoptotic cells were significantly higher in Cx43 overexpressing cells compared with cells transfected with the EV (**Figure 1f**). These results together indicate that Cx43 reduces cell proliferation and enhances senescence and cell death by apoptosis in BRAF mutant melanoma cells (A375). Deregulation of the MAPK pathway is mainly caused by mutations in BRAF and in NRAS. In order to investigate the role of Cx43 in NRAS mutant cell lines, we transfected the melanoma cell lines Sk-Mel-147 with a NRAS mutation with the vector to overexpress Cx43. In **Figure 1g** we observed that this cell line forms smaller colonies and shows lower levels of cell when Cx43 is overexpressed. In order to confirm the role of Cx43 in other cancers with a mutation in BRAF, we transfected different human cell lines from colorectal and breast cancer with a mutation in BRAF with the Cx43 overexpressing vector. The overexpression of Cx43 in both tumour cell lines significantly reduced cell proliferation and the capacity to form colonies (**Figure 1h**). These results demonstrated that Cx43 acts as a tumour suppressor reducing cellular proliferation in BRAF and NRAS mutant tumour cells. However, the role of Cx43 in reducing proliferation and increasing senescence and apoptosis seems to occur independently of its channel activity as shown in **Figure 1d****.** As Cx43 was found in EVs including exosomes and these particles has been involved in tumour progression, we have decided to investigate the role of Cx43 within these vesicles.

### Example 2.2. Cx43 levels are found in extracellular vesicles (EVs) including exosomes.

EVs including exosomes have been reported to be involved in the development and progression of cancer malignancy by promoting cancer proliferation, establishing a premetastatic niche, and regulating drug resistance. We have isolated EVs from A375 cell line and have characterized them by electron microscopy and by nanoparticle tracking analysis (NTA) confirming the sizes of these particles are between 20-100 nm (**Figure 2a**). The presence of Cx43 was studied by western blot and we detected that cells that overexpress Cx43 contain this protein in their EVs (**Figure 2b**). The tetraspanin EVs marker CD9 was used as a control. Same results were confirmed in other tumour cell lines transfected with the same vector to overexpress Cx43 (**Figure 2c**).

### Example 2.3. The presence of Cx43 in EVs changes the protein and small RNA content.

Cx43 can interact with different proteins and probably with different molecules of RNA and DNA. For this reason, we have decided to investigate if the presence of Cx43 in the EVs could change the protein and RNA content of the EVs secreted by tumour cells that overexpress this channel protein. EVs have the ability to carry cell-specific cargos of proteins, lipids, and genetic materials, and can be selectively taken up by neighbouring or distant cells and modify their signalling and functions. In order to study if the presence of Cx43 in EVs could affect their content, we performed a proteomic (LC-MS/MS) and RNA sequence analysis. To determine the protein profile of EVs (Cx43 negative (-) and Cx43 positive (+) EVs), total exosomal proteins were separated by SDS-PAGE. A total of 1141 proteins were identified for both groups of EVs (Cx43 - and Cx43 +), 160 proteins were exclusive of EVs negative for (Cx43 -) and 482 proteins were exclusive for EVs containing (Cx43 +) (**Figure 3a**). These proteins were represented in a Proteomap (**Figure 3b**), where it is shown the KEGG Orthology and the biological process terms associated with low and high abundance of proteins observed in EVs Cx43 - and in EVs Cx43 +. Gene set enrichment analysis (GSEA), Gene Ontology (GO), KEGG Orthology and Reactome data base were used to identify pathways and biological processes significantly enriched in EVs isolated from Cx43 - (**Figure 3c**, orange) and Cx43 + (**Figure 3c**, blue). We observed a significant increment in some of the identified proteins in EVs containing Cx43 by enrichment analysis (see normalized enrichment score (NES) and p-values, **Figure 3c**). The proteomic results indicate that the presence of Cx43 increases the number of proteins that are recruited to the EVs (482 proteins in EVs Cx43 + and 160 proteins in EVs Cx43 -) and also radically changes the protein content which is now enriched in pathways such as nuclear acid binding, DNA repair, mitochondrial matrix, RNA binding, regulation of cell motility, regulation of cytokine production or regulation of cell migration (**Figure 3d**) These results demonstrate that Cx43 radically changes the protein content of EVs. Proteins such as CELF1, CPSF2 (involved in RNA splicing); DCTN2, FAM105B (immune system processes); SMARCA4, SMARCC1 (chromatin disassembly); MCM7, CDK1, CDK6 (G1/S transition of mitotic cell cycle); LIMS1, MAP2K1(cell aging) or MGEA5, PGAM5 (necrotic cell death), were only found in EVs containing Cx43.

On the other hand, enrichment analysis of small RNA content was analysed using MiRNet 2.0. Heatmaps show pathways and biological processes significantly enriched in EVs isolated from Cx43 - (**Figure 3e**, blue) and Cx43 + (**Figure 3e**, green). Pathways are classified according to the number of genes that are enriched in each of them (observed gene counts) and its FDR. We observed several small RNAs involved in pathways such as cell dead, autophagy and cell cycle arrest in EVs containing Cx43.

The presence of Cx43 also changes the patterns of small RNAs that modify cellular function in recipient cells. RNA sequencing analysis showed (**Figure 3f**) a radical change in the types of RNA that are transport in these vesicles depending on the presence of Cx43. EVs containing Cx43 are mainly enriched in miRNAs and rRNAs which represents the 28.2% and 20.2% of all raw reads, respectively. In EVs negative for Cx43 the most abundant RNAs were tRNA and miRNAs such as hsa-mir-24-2, hsa-miR-34a-5p, hsa-mir-30c-2, hsa-miR-140-5p, hsa-miR-19a-3p and hsa-miR-125b-5p were upregulated for more than 400 folds in Cx43 positive EVs compared with Cx43 negative EVs with differential fold changes between a range of 448.77 (hsa-miR-125b-5p) and 8550.33 (hsa-mir-24-2) (**Figure 3g**). This miRNAs are involved in process such as negative regulation of cell proliferation (hsa-mir-30c-2, hsa-mir-24-2), migration (hsa-mir-24-2, has-miR-34a-5p, hsa-mir-30c-2), angiogenesis hsa-miR-34a-5p, hsa-miR-125b-5p) and positive regulation of cell death (hsa-miR-34a-5p). When we analysed the relative abundances of transcripts and fold changes between Cx43 negative and Cx43 positive EVs and we have detected several small RNA overrepresented in Cx43 positive EVs, such as an enrichment of miRNAs (shown in **Figure 3g**), piRNAs (e.g piR-30112, piR-31447 or piR-31703), CDBox (HBII-2020, U58A) or HacaBox (ACA3, HBI-6 or ACA53) (**Figure 3h**). These data suggest that the presence of Cx43 in EVs changes the content of small RNAs and proteins, probably changes their message and signalling in target cells.

### Example 2.4. The presence of Cx43 in EVs switches the role of these vesicles promoting the anti-tumour activity.

In order to investigate the role of Cx43 in EVs, tumour cells were treated with EVs containing or not Cx43 and released by melanoma cells overexpressing Cx43 or by normal melanoma cells which hardly express Cx43 (**Figure 4a**). The presence of Cx43 in the EVs from donor cells (**Figure 4a**) was confirmed by western-blot (cell lysate and EVs) and the capacity to enter in the target cells was studied by IF (EVs were labelled with DIL and detected within the receptor cells by fluorescent microscopy) (**Figure 4b**). To study the effect of these EVs, two different tumour cell lines (A375 and BLM) were used as recipient cells in colony formation assays. Both cell lines were treated with EVs from A375-EV and A375-Cx43. As it is shown in **Figure 4c**, we have observed a significant reduction in the capacity to form colonies and cell proliferation when tumour cells were treated with EVs containing Cx43. The treatment with EVs positives for Cx43 also upregulated Cx43 protein and mRNA levels in target cells and detected by western blotting and RT-qPCR (**Figure 4d**).

### Example 2.5. Cx43 levels increase when tumour cells are treated with BRAF/MEK inhibitors.

Targeting BRAF and MEK using specific inhibitors have become the standard of care for patients with melanoma and tumours with a mutation in BRAF. However, the therapeutic benefits are often limited due to the development of drug resistance. To examine the potential effect of Cx43 on the response of tumour cells to BRAF/MEK inhibitors (BRAF/MEKi) (Dabrafenib/Trametinib) treatment, A375 melanoma cell line was treated with Dabrafenib and Trametinib (**Figure 5a**). During treatments we have observed by RT-qPCR and western-blot an increase in the levels of Cx43 in A375 cell line when these cells were sensible to treatments (**Figure 5a**). Further, when Cx43 was overexpressed using a vector, tumour cells respond more efficiently to different concentrations of BRAF/MEKi by decreasing cellular proliferation and the capacity to form colonies (**Figure 5b**, left). The presence of Cx43 under treatments enhanced cellular senescence detected by flow cytometry and by β-Gal Staining (**Figure 5c**). To confirm that now cells are more sensible to apoptosis, we have performed a western blot analysis to detect the cleavage of caspase-3 (Figure 5f). Cx43 increases cellular senescence and apoptosis in tumour cells under these treatments. In order to test if the combination with a senolytic molecule could increase the efficacy of treatments, we have combined the BRAF/MEKi with Cx43 overexpression and navitoclax (NAV). As it is shown in Figure 5f, cells that overexpressed Cx43 in combination with BRAF/MEKi (red box) and Cx43 in combination with BRAF/MEKi plus NAV, showed the highest levels of cleavage caspase-3 (green box) indicating that the combination results in a more efficient treatment to activate cell death of tumour cells under BRAF/MEKi treatment (Figure 5f).

### Example 2.6. Resistant A375 tumour cells to BRAF/MEKi loss Cx43 acquire a mesenchymal-like phenotype.

To test whether Cx43 is also involved in acquired resistance of melanoma cell to BRAF/MEKi, cells were selected for resistance to Dabrafenib and Trametinib by prolongated exposure to high concentrations of these drugs. After 4 months, double resistant cells (DR) hardly responded to high concentrations of both drugs (**Figure 6a**). When cells were DR to BRAF/MEKi, Cx43 protein was downregulated (**Figure 6b**) in comparison with their parental counterpart and with cells sensible to treatments (S). We also observed a decrease in Cx43 mRNA levels (**Figure 6b**). DR cells downregulated Cx43 and underwent a phenotypic change that corresponded to an epithelial to mesenchymal transition (EMT) as detected by western blotting (**Figure 6c**). The EMT transcription factor Twist-1 was shown upregulated in DR cells and localized in the nuclear fraction. The increase in Twist-1 was accompanied by elevated levels of EMT marker N-Cadherin in the cytoplasm (**Figure 6c**) and correlates with a loss of Cx43. The next point was to see how the overexpression of Cx43 affected the resistance to BRAF/MEKi at different times (1 week, 2 week and 4 months). Interestingly, when cells were grown in the presence of Cx43 for 1 and 2 weeks and for 4 months, the colony formation assays (**Figure 6d**) showed that overexpression of Cx43 avoid the acquirement of drug resistance (**Figure 6d**). Furthermore, when BRAF/MEKi resistant cells (DR) were transfected with a vector to overexpress the protein, DR cells became sensible to treatments and the Cx43 re-expression in DR cells increases the MFI ratio of apoptotic cells (**Figure 6e**).

### Example 2.7. EVs containing high levels of Cx43 have anti-tumour activity and improve BRAF/MEKi efficacy.

When we treated melanoma tumour cells (A375) with BRAF/MEKi we detected an increase in Cx43 protein levels when cells are sensible to these treatments (**Figure 7a**). The treatment with both inhibitors also increases Cx43 protein levels in the EVs released by these cells, (**Figure 7a**) indicating that now, these particles could have different function (**Figure 4c**). In order to study the effect of Cx43 within EVs, we isolated EVs from HEK 293 cell line which contain high levels of Cx43 as it is shown by western blotting in **Figure 7b****.** To confirm that EVs positives for Cx43 has an anti-proliferative effect, A375 cells were treated with HEK 293 derived-EVs (**Figure 7c**). When A375 cells were treated with EVs positives for Cx43, we observed a reduction in colony formation and an increase in the efficacy of BRAF/MEKi treatment, when cells were co-treated with EVs positive for Cx43 and with BRAF/MEKi (**Figure 7c**). Further, when we treat double resistant cells with BRAF/MEKi (DR) with EVs containing Cx43, we observed a decrease in the capacity to form colonies, and importantly, these DR cells now are more sensible to BRAF/MEKi treatments (**Figure 7d**). These inhibitors increase cellular senescence and this stage of cell cycle arrest has been involved in drug resistance and tumour progression. In fact, the combination with a senolytic drug that specifically target senescent cells, such as navitoclax (NAV), is currently under clinical trials in USA (ClinicalTrials.gov Identifier: NCT01989585). Our results indicate that the combination of NAV with EVs containing Cx43, significantly reduce the capacity to form colonies of tumour cells (**Figure 7e**). Importantly, the combination of EVs positives for Cx43 with NAV and BRAF/MEKi, effectively target DR cells, significantly decreasing their capacity to form colonies (**Figure 7f**).

These results demonstrate that Cx43 within EVs results in (i) an effective therapeutic strategy to reduce cellular proliferation in BRAF-mutant tumour cells; (ii) increases the efficacy of BRAF/MEKi and (iii) the combination with navitoclax and BRAF/MEKi targets resistant tumour cells re-sensitive DR cells to BRAF/MEKi (**Figure 7c****-f**) avoiding drug resistance.

## Claims

1. Connexin 43 for use in the treatment of cancer, wherein the cancer is **characterized by** the activation of a mitogen-activated protein kinase (MAPK) selected from the list consisting of: BRAF, RAS, MEK or ERK.

2. Connexin 43 for use, according to claim 1, in the treatment of cancer wherein the cancer is **characterized by** the activation of BRAF or NRAS.

3. Connexin 43 for use, according to any of the previous claims, in the treatment of a type of cancer selected from: melanoma, colon cancer, lung cancer or breast cancer.

4. Connexin 43 for use, according to any of the previous claims, before, after or simultaneously to a treatment with an inhibitor of a mitogen-activated protein kinase (MAPK) selected from the list consisting of: BRAF, RAS, MEK or ERK.

5. Connexin 43 for use, according to any of the previous claims, before, after or simultaneously to a treatment with a BRAF and/or MEK inhibitor.

6. Connexin 43 for use, according to any of the previous claims, before, after or simultaneously to a treatment with dabrafenib, trametinib, vemurafenib, encorafenib, cobimetinib and/or binimetinib.

7. Connexin 43 for use, according to any of the previous claims, before, after or simultaneously to a treatment with a senolytic agent, preferably navitoclax.

8. Combination drug product comprising Connexin 43 and an inhibitor of a mitogen-activated protein kinase (MAPK) selected from the list consisting of: BRAF, RAS, MEK or ERK.

9. Combination drug product, according to claim 8, comprising connexin 43 and a BRAF and/or MEK inhibitor.

10. Combination drug product, according to any of the claims 8 or 9, comprising connexin 43 and dabrafenib, trametinib, vemurafenib, encorafenib, cobimetinib and/or binimetinib.

11. Combination drug product, according to any of the claims 8 to 10, further comprising a senolytic agent, preferably navitoclax.

12. Pharmaceutical composition comprising the combination drug product of any of the claims 8 to 11 and, optionally, pharmaceutically acceptable excipients or carriers.

13. Pharmaceutical composition for use, according to claim 12, in the treatment of cancer wherein the cancer is **characterized by** the activation of mitogen-activated protein kinase (MAPK) selected from the list consisting of: BRAF, RAS, MEK or ERK wherein Connexin 43 is administered by using a delivery vehicle.

14. Pharmaceutical composition for use, according to claim 13, wherein the delivery vehicle is a nanoparticle, an extracellular vesicle or an expression vector which encodes Connexin 43.

## Patentansprüche

1. Connexin 43 zur Verwendung in der Behandlung von Krebs, wobei der Krebs durch die Aktivierung einer mitogenaktivierten Proteinkinase (MAPK) ausgewählt aus der Liste bestehend aus: BRAF, RAS, MEK oder ERK, charakterisiert ist.

2. Connexin 43 zur Verwendung, nach Anspruch 1, in der Behandlung von Krebs, wobei der Krebs durch die Aktivierung von BRAF oder NRAS charakterisiert ist.

3. Connexin 43 zur Verwendung, nach einem der vorhergehenden Ansprüche, in der Behandlung eines Krebstyps ausgewählt aus: Melanom, Dickdarmkrebs, Lungenkrebs oder Brustkrebs.

4. Connexin 43 zur Verwendung, nach einem der vorhergehenden Ansprüche, vor, nach oder gleichzeitig zu einer Behandlung mit einem Inhibitor einer mitogenaktivierten Proteinkinase (MAPK) ausgewählt aus der Liste bestehend aus: BRAF, RAS, MEK oder ERK.

5. Connexin 43 zur Verwendung, nach einem der vorhergehenden Ansprüche, vor, nach oder gleichzeitig zu einer Behandlung mit einem BRAF- und/oder MEK-Inhibitor.

6. Connexin 43 zur Verwendung, nach einem der vorhergehenden Ansprüche, vor, nach oder gleichzeitig zu einer Behandlung mit Dabrafenib, Trametinib, Vemurafenib, Encorafenib, Cobimetinib und/oder Binimetinib.

7. Connexin 43 zur Verwendung, nach einem der vorhergehenden Ansprüche, vor, nach oder gleichzeitig zu einer Behandlung mit einem Senolytikum, vorzugsweise Navitoclax.

8. Kombinationsarzneimittel umfassend Connexin 43 und einen Inhibitor einer mitogenaktivierten Proteinkinase (MAPK) ausgewählt aus der Liste bestehend aus: BRAF, RAS, MEK oder ERK.

9. Kombinationsarzneimittel nach Anspruch 8, umfassend Connexin 43 und einen BRAF- und/oder MEK-Inhibitor.

10. Kombinationsarzneimittel nach einem der Ansprüche 8 oder 9, umfassend Connexin 43 und Dabrafenib, Trametinib, Vemurafenib, Encorafenib, Cobimetinib und/oder Binimetinib.

11. Kombinationsarzneimittel nach einem der Ansprüche 8 bis 10, zusätzlich umfassend ein Senolytikum, vorzugsweise Navitoclax.

12. Pharmazeutische Zusammensetzung umfassend das Kombinationsarzneimittel nach einem der Ansprüche 8 bis 11 und, wahlweise, pharmazeutisch akzeptable Hilfsstoffe oder Träger.

13. Pharmazeutische Zusammensetzung zur Verwendung, nach Anspruch 12, in der Behandlung von Krebs, wobei der Krebs durch die Aktivierung einer mitogenaktivierten Proteinkinase (MAPK) ausgewählt aus der Liste bestehend aus: BRAF, RAS, MEK oder ERK charakterisiert ist, wobei Connexin 43 unter Verwendung eines Transportvehikels verabreicht wird.

14. Pharmazeutische Zusammensetzung zur Verwendung, nach Anspruch 13, wobei das Transportvehikel eine Nanopartikel, eine extrazelluläre Vesikel oder ein Expressionsvektor, welcher Connexin 43 kodiert, ist.

## Revendications

1. Connexine 43 destinée à être utilisée dans le traitement de cancer, dans laquelle le cancer est **caractérisé par** l'activation d'une protéine kinase activée par mitogène (MAPK) sélectionnée parmi la liste consistant en : BRAF, RAS, MEK ou ERK.

2. Connexine 43 destinée à être utilisée, selon la revendication 1, dans le traitement de cancer dans laquelle le cancer est **caractérisé par** l'activation de BRAF ou NRAS.

3. Connexine 43 destinée à être utilisée, selon l'une quelconque des revendications précédentes, dans le traitement d'un type de cancer sélectionné parmi : le mélanome, le cancer du côlon, le cancer du poumon ou le cancer du sein.

4. Connexine 43 destinée à être utilisée, selon l'une quelconque des revendications précédentes, avant, après ou simultanément à un traitement avec un inhibiteur d'une protéine kinase activée par mitogène (MAPK) sélectionnée parmi la liste consistant en : BRAF, RAS, MEK ou ERK.

5. Connexine 43 destinée à être utilisée, selon l'une quelconque des revendications précédentes, avant, après ou simultanément à un traitement avec un inhibiteur de BRAF et/ou de MEK.

6. Connexine 43 destinée à être utilisée, selon l'une quelconque des revendications précédentes, avant, après ou simultanément à un traitement avec dabrafenib, trametinib, vemurafenib, encorafenib, cobimetinib et/ou binimetinib.

7. Connexine 43 destinée à être utilisée, selon l'une quelconque des revendications précédentes, avant, après ou simultanément à un traitement avec un agent sénolytique, préférablement navitoclax.

8. Produit pharmaceutique de combinaison comprenant connexine 43 et un inhibiteur d'une protéine kinase activée par mitogène (MAPK) sélectionnée parmi la liste consistant en : BRAF, RAS, MEK ou ERK.

9. Produit pharmaceutique de combinaison, selon la revendication 8, comprenant connexine 43 et un inhibiteur de BRAF et/ou de MEK.

10. Produit pharmaceutique de combinaison, selon l'une quelconque des revendications 8 ou 9, comprenant connexine 43 et dabrafenib, trametinib, vemurafenib, encorafenib, cobimetinib et/ou binimetinib.

11. Produit pharmaceutique de combinaison, selon l'une quelconque des revendications 8 à 10, comprenant en outre un agent sénolytique, préférablement navitoclax.

12. Composition pharmaceutique comprenant le produit pharmaceutique de combinaison selon l'une quelconque des revendications 8 à 11 et, optionnellement, des excipients ou véhicules pharmaceutiquement acceptables.

13. Composition pharmaceutique destinée à être utilisée, selon la revendication 12, dans le traitement de cancer dans laquelle le cancer est **caractérisé par** l'activation d'une protéine kinase activée par mitogène (MAPK) sélectionnée parmi la liste consistant en : BRAF, RAS, MEK ou ERK, dans laquelle la connexine 43 est administrée en utilisant un véhicule d'administration

14. Composition pharmaceutique destinée à être utilisée, selon la revendication 13, dans laquelle le véhicule d'administration est une nanoparticule, une vésicule extracellulaire ou un vecteur d'expression qui code la connexine 43.
